# EUROPEAN PATENT SPECIFICATION

(11) **EP 0 604 798 B1**
(45) Date of publication and mention of the grant of the patent: **20.02.2002**
(21) Application number: 93119754.5
(22) Date of filing: 08.12.1993
(51) Int. Cl.: A01N 37/28, A01N 37/52, A01N 43/40, A01N 43/58, C07C 257/22

(54) **N-arylhydrazine derivatives as insecticidal and acaricidal agents**
N-Arylhydrazinderivate als insektizide und akarizide Mittel
Dérivés de la N-arylhydrazine en tant qu'agents insecticides et acaricides

(30) Priority: 29.12.1992 US 998105; 29.12.1992 US 998104; 29.12.1992 US 998101
(43) Date of publication of application: 06.07.1994
(73) Proprietor: BASF AKTIENGESELLSCHAFT, 67056 Ludwigshafen (DE)
(72) Inventor: Furch, Joseph Augustus, Lawrenceville, New Jersey (US); Kuhn, David George, Newton, Pennsylvania (US); Hunt, David Allen, Newton, Pennsylvania (US); Lew, Albert Chieh, Princeton Junction, New Jersey 08850 (US); Gronostajski, Cynthia Emma, Trenton, New Jersey 08611 (US)

(56) References cited:
- EP-A- 0 325 983
- DE-A- 4 200 591
- FR-A- 2 105 698
- FR-A- 2 184 974
- GB-A- 736 473
- US-A- 3 214 334
- US-A- 3 505 403
- US-A- 3 745 215
- US-A- 3 879 542
- US-A- 3 917 849
- US-A- 3 935 315
- JOURNAL OF ORGANIC CHEMISTRY vol. 38, no. 7 , 1973 , EASTON US pages 1344 - 1348 R.F.SMITH ET AL. 'Amidrazones'
- JOURNAL OF THE CHEMICAL SOCIETY, PERKIN TRANSACTIONS 2 1986 , LETCHWORTH GB pages 537 - 541 I.D.CUNNINGHAM ET AL. 'Acid, base, and uncatalysed isomerisation of z- to e- amidine'
- BULLETIN DE LA SOCIETE CHIMIQUE DE FRANCE no. 1 , 1971 , PARIS FR pages 283 - 286 J.P. CHAPELLE ET AL. 'Recherches sur les ènehydrazines'
- CHEMICAL ABSTRACTS, vol. 92, no. 21, 26 May 1980, Columbus, Ohio, US; abstract no. 180607j, & ZH. ORG. KHIM. vol. 15, no. 11 , 1979 pages 2280 - 2287

## Description

### BACKGROUND OF THE INVENTION

Certain insect and acarid pests are harmful and cause enormous losses annually in agricultural crops, stored products and human and animal health. GB-A-736473 discloses a pesticidal composition comprising a hydrazine derivative. EP-A-325983 discloses N-phenylbenzamides and N-phenylbenzamide oximes, methods for their production, compositions containing them, and their use as pesticides. US-A-3214334 and FR-A-2184974 disclose phenyl hydrazine derivatives suitable as fungicides. J. Org. Chem., Vol. 38, No. 7, 1973, pp. 1344-1348 discloses phenylhydrazone derivatives.

It is an object of this invention to provide substituted N-arylhydrazine derivatives which are effective agents for the control of pestiferous insects and acarina.

It is another object of this invention to provide a method for the protection of important agronomic crops from the harmful and damaging effects caused by insect and acarid pests.

It is a further object of this invention to provide insecticidal and acaricidal compositions, and their use for controlling insect or acarid pests.

### SUMMARY OF THE INVENTION

The present invention provides a method for the control of insects or acarina which comprises contacting said insects or acarina or their food supply, breeding ground or habitat with an insecticidally effective amount of an N-arylhydrazine derivative of formula I 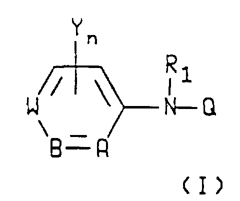 wherein
- A: is C-R₄ or N;
- B: is C-R₅ or N;
- W: is C-R₆ or N with the proviso that one of A, B or W must be other than N; and with the further proviso that when all of A, B, and W are other than N, then R cannot be phenyl or substituted phenyl;
- Y: is hydrogen, halogen, CN, NO₂, C₁-C₆alkyl, C₁-C₆haloalkyl, C₁-C₆alkoxy or C₁-C₆haloalkoxy;
- n: is an integer of 0, 1 or 2;
- Q: is 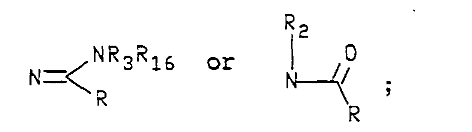
- R: is hydrogen, C₁-C₁₀alkyl optionally substituted with one or more halogens, C₃-C₆ cycloalkyl, C₁-C₄alkoxy, C₁-C₄haloalkoxy, (C₁-C₄alkyl)SOₓ, (C₁-C₄haloalkyl)SOₓ, phenyl optionally substituted with one to three halogen, C₁-C₄alkyl, C₁-C₄haloalkyl, C₁-C₄alkoxy, C₁-C₄haloalkoxy, (C₁-C₄alkyl)SOₓ, (C₁-C₄haloalkyl)SOₓ, NO₂ or CN groups, or
phenoxy optionally substituted with one to three halogen, C₁-C₄alkyl, C₁-C₄haloalkyl, C₁-C₄alkoxy, C₁-C₄haloalkoxy, (C₁-C₄alkyl)SOₓ, (C₁-C₄haloalkyl)SOₓ, NO₂ or CN groups,
C₃-C₁₂ cycloalkyl optionally substituted with one or more halogens, C₁-C₆alkyl, C₁-C₆haloalkyl, C₁-C₄alkoxy, C₁-C₄-haloalkoxy, (C₁-C₄alkyl)SOₓ, (C₁-C₄haloalkyl)SOₓ, phenyl optionally substituted with one to three halogen, C₁-C₄alkyl, C₁-C₄haloalkyl, C₁-C₄alkoxy, C₁-C₄haloalkoxy, NO₂ or CN groups, or phenoxy optionally substituted with one to three halogen, C₁-C₄alkyl, C₁-C₄haloalkyl, C₁-C₄alkoxy, C₁-C₄-haloalkoxy, NO₂ or CN groups, or
phenyl optionally substituted with one or more halogen, C₁-C₄alkyl, C₁-C₄haloalkyl, C₁-C₄alkoxy, C₁-C₄haloalkoxy, NO₂ or CN groups;
- R₁ and R₂: are each independently hydrogen or C₁-C₄alkyl;
- R₃ and R₁₆: are each independently hydrogen, C₁-C₁₀alkyl optionally substituted with one or more halogen, hydroxy, C₁-C₄alkoxy, (C₁-C₄alkyl)SOₓ, CONR₇R₈, CO₂R₉, R₁₀, R₁₁, C₃-C₆cycloalkyl optionally substituted with one to three halogen, C₁-C₄alkyl, C₁-C₄haloalkyl, C₁-C₄-alkoxy, C₁-C₄haloalkoxy, NO₂ or CN groups,
phenyl optionally substituted with one or more halogen, C₁-C₄alkyl, C₁-C₄haloalkyl, C₁-C₄alkoxy, C₁-C₄haloalkoxy, CO₂ or CN groups, or pyridyl optionally substituted with one or more halogen, C₁-C₄alkyl, C₁-C₄haloalkyl, C₁-C₄alkoxy, C₁-C₄-haloalkoxy, NO₂ or CN groups,
C₃-C₁₀alkenyl optionally substituted with one or more halogen, hydroxy, C₁-C₄-alkoxy, (C₁-C₄alkyl)SOₓ, CONR₇R₈, CO₂R₉, R₁₀, R₁₁, C₃-C₆cycloalkyl optionally substituted with one to three halogen, C₁-C₄alkyl, C₁-C₄haloalkyl, C₁-C₄alkoxy, C₁-C₄haloalkoxy, NO₂ or CN groups,
phenyl optionally substituted with one or more halogen, C₁-C₄alkyl, C₁-C₄haloalkyl, C₁-C₄alkoxy, C₁-C₄-haloalkoxy, CO₂ or CN groups, or pyridyl optionally substituted with one or more halogen, C₁-C₄alkyl, C₁-C₄haloalkyl, C₁-C₄alkoxy, C₁-C₄-haloalkoxy, NO₂ or CN groups,
C₃-C₁₀alkynyl optionally substituted with one or more halogen, hydroxy, C₁-C₄-alkoxy, (C₁-C₄alkyl)SOₓ, CONR₇R₈, CO₂R₉, R₁₀, R₁₁, C₃-C₆cycloalkyl optionally substituted with one to three halogen, C₁-C₄alkyl, C₁-C₄haloalkyl, C₁-C₄alkoxy, C₁-C₄ haloalkoxy, NO₂ or CN groups,
phenyl optionally substituted with one or more halogen, C₁-C₄alkyl, C₁-C₄haloalkyl, C₁-C₄alkoxy, C₁-C₄-haloalkoxy, or CN groups, or pyridyl optionally substituted with one 1 or more halogen, C₁-C₄alkyl, C₁-C₄haloalkyl, C₁-C₄alkoxy, C₁-C₄-haloalkoxy, NO₂ or CN groups,
C₃-C₁₂cycloalkyl optionally substituted with one or more halogen, hydroxy, C₁-C₄-alkoxy, (C₁-C₄alkyl)SOₓ, CONR₇R₈, CO₂R₉, R₁₀, R₁₁, C₃-C₆cycloalkyl optionally substituted with one to three halogen, C₁-C₄alkyl, C₁-C₄haloalkyl, C₁-C₄alkoxy, C₁-C₄-haloalkoxy, NO₂ or CN groups, phenyl optionally substituted with one or more halogen, C₁-C₄alkyl, C₁-C₄haloalkyl, C₁-C₄alkoxy, C₁-C₄-haloalkoxy, or CN groups, or pyridyl optionally substituted with one or more halogen, C₁-C₄alkyl, C₁-C₄haloalkyl, C₁-C₄alkoxy, C₁-C₄-haloalkoxy, NO₂ or CN groups or
- R₃ and R₁₆: may be taken together to form a ring represented by the structure 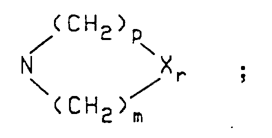
- R₄, R₅ and R₆: are each independently hydrogen, halogen, CN, NO₂, (C₁-C₄alkyl)SOₓ, (C₁-C₄-haloalkyl)SOₓ, C₁-C₆alkyl, C₁-C₆haloalkyl, C₁-C₆alkoxy or C₁-C₆haloalkoxy;
- R₇, R₈ and R₉: are each independently hydrogen or C₁-C₄ alkyl;
- R₁₀: is NR₁₂R₁₃, 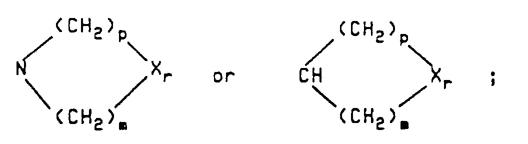
- R₁₁: is 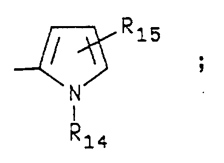
- R₁₂, R₁₃, R₁₄ and R₁₅: are each independently hydrogen or C₁-C₄alkyl;
- X: is O, S or NR₁₄;
- r: is an integer of 0 or 1;
- p and m: are each independently an integer of 0, 1, 2 or 3 with the provisos that only one of p, m or r can be 0 and that the sum of p + m + r must be 4, 5 or 6;
- x: is an integer of 0, 1 or 2; or
the acid addition salts thereof.

The present invention further provides N-arylamidrazone compounds of formula I wherein A, B, W, Y, n, and R₁, are as described hereinabove and Q is 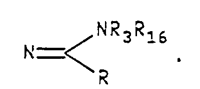 with the proviso that when all of A, B and W are other than N, then one of R₄ or R₆ must be other than NO₂ and R₄ and at least one of R₃ or R₁₆ must be other than hydrogen and with the further proviso that when one of A, B or W is N, then at least one of Y, R₄, R₅, and R₆ must be other than C₁-C₁₀alkyl.

Compositions and methods for the protection of growing plants from attack and infestation by insects and acarina are also provided.

### DETAILED DESCRIPTION OF THE INVENTION

A variety of insects and acarina cause great economic loss by damaging or destroying agricultural crops and other valuable plants; by aiding in the spread and development of bacteria, fungi and viruses that produce diseases of plants; and by destroying or lowering the value of stored foods, other products and possessions. Insects and acarina present some of the farmers' greatest problems the world over. The need for alternative and effective insect and acarid control is a global concern.

It has now been found that the substituted N-arylhydrazone derivatives of formula I are especially efficacious insecticidal and acaricidal agents, particularly against Coleoptera, Lepidoptera and Acarina.

The formula Ia amidrazone compounds of the present invention have the structural formula 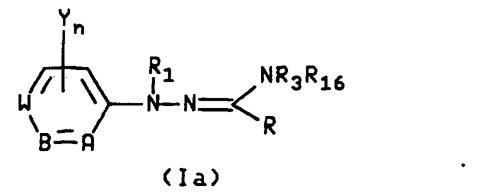 wherein A, B, W, Y, n, R, R₁, R₃ and R₁₆ are described hereinabove. The term halogen as used in the specification and claims designates chlorine, fluorine, bromine or iodine. The term acid addition salts designates those salts formed by acids commonly known in the art such as hydrogen chloride, hydrogen bromide, hydrogen bisulfate, hemi-hydrogen sulfate and the like. In the above definition when n is 0 then Y is hydrogen.

Preferred compounds of the invention are those wherein R, R₃ and R₁₆ are each independently hydrogen or C₁-C₆alkyl, A is C-R₄, B is C-R₅, W is C-R₆, Y is halogen and n is 1. Particularly preferred compounds are those wherein R₁ is hydrogen, R₄ is halogen, R₅ is hydrogen and/or R₆ is C₁-C₆alkyl substituted with one or more halogens, preferably trifluoromethyl.

Other preferred compounds of the invention are compounds having the structure 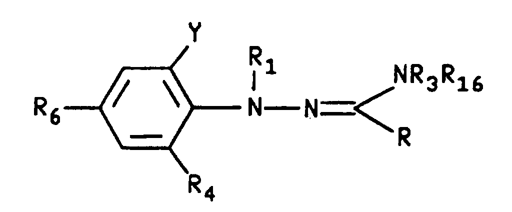 wherein
- R: is C₁-C₁₀alkyl;
- R₁: is hydrogen or C₁-C₄alkyl;
- R₃: is C₁-C₁₀alkyl;
- R₁₆: is hydrogen or C₁-C₁₀alkyl; and
- R₄, R₆ and Y: are each independently hydrogen, halogen, CN, NO₂, C₁-C₆alkyl, C₁-C₆ haloalkyl, C₁-C₆ alkoxy, or C₁-C₆ haloalkoxy.

The N-arylamidrazones of formula Ia may be prepared by reacting an acid chloride, hydrazone (hydrazinoyl chloride) of formula II with an amine compound, HNR₃R₁₆, as shown in flow diagram I.

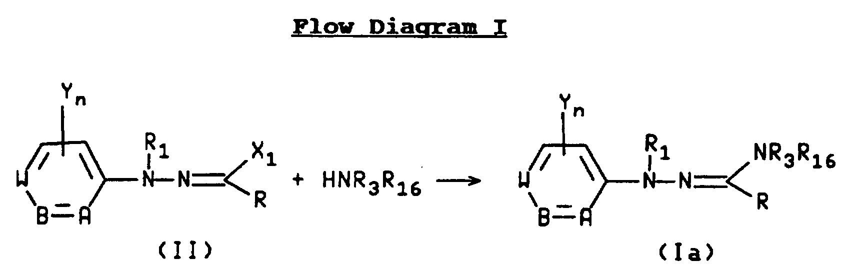

Compounds of formula II may be prepared by reacting a suitable arylhydrazine of formula III with the appropriate acid chloride, RCOCl, to obtain an N-arylhydrazide of formula IV and reacting the formula IV hydrazide with a halogenating agent such as thionyl halide to give the desired formula II N-arylhydrazinoyl halide product. The reaction is illustrated in flow diagram II.

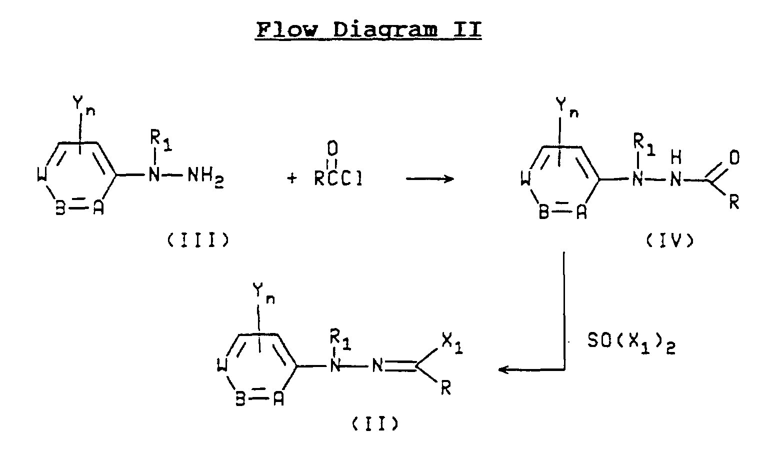

The substituted N-arylhydrazine derivatives of the present invention are effective for controlling insect and acarid pests. Said compounds are also effective for protecting growing or harvested crops from attack and infestation by such pests.

Compounds useful include N-arylhydrazinoyl halide compounds of formula II. The insecticidal and acaricidal formula II hydrazinoyl halides have the structural formula 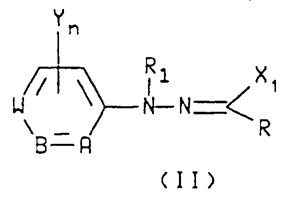 wherein A, B, W, Y, n, R, R₁ and X₁ are described hereinabove.

Compounds of formula II are those compounds wherein R₁ is hydrogen, A is C-R₄, B is C-R₅, W is C-R₆, Y is halogen or nitro and n is 1.

Other compounds of formula II are those in which R is optionally substituted C₃-C₁₂ cycloalkyl or C₁-C₁₀ haloalkyl, preferably C₁-C₆ haloalkyl.

Compounds of formula II wherein X₁ is fluorine may be prepared from compounds of formula II wherein X₁ is chlorine or bromine by a halogen exchange reaction using sodium fluoride or hydrogen fluoride such as that described by March in Advanced Organic Chemistry, 4 Ed. (1992), p. 438.

Further compounds useful in the method of invention include substituted carboxylic acid, N-arylhydrazide compounds of formula V.

The insecticidal and acaricidal formula V N-arylhydrazides of the present invention have the structural formula

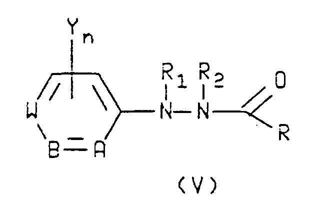

Preferred compounds of formula V for use in the method of the invention are those compounds wherein R is hydrogen or C₁-C₆alkyl, A is C-R₄, B is C-R₅, W is C-R₆, Y is halogen or nitro and n is 1. Particularly preferred formula V N-arylhydrazides are those wherein R₄ is halogen, R₅ is hydrogen and R₆ is C₁-C₆alkyl substituted with one or more halogens, preferably trifluoromethyl.

Compounds of formula V may be prepared by reacting a suitable arylhydrazine of formula VI with the appropriate acid chloride, RCOCl, to yield the desired N-arylhydrazide of formula V. The reaction is illustrated in flow diagram III.

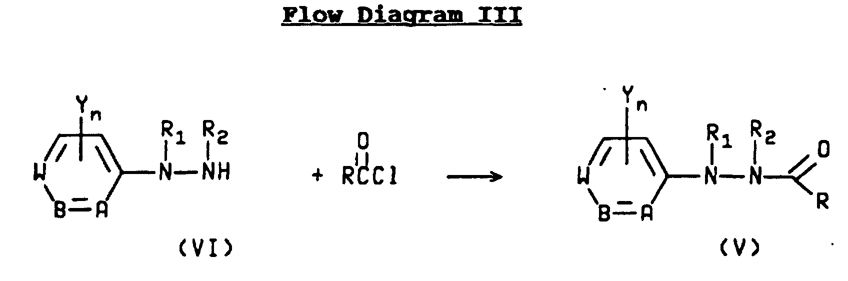

Growing or harvested crops may be protected from attack or infestation by insect or acarid pests by applying to the foliage of the crops, or to the soil or water in which they are growing, a pesticidally effective amount of a formula I N-arylhydrazine derivative.

In practice, generally about 10 ppm to 10,000 ppm, preferably about 100 to 5,000 ppm of the formula I compound dispersed in a liquid carrier, when applied to the plants or the soil or water in which they are growing, is effective to protect the plants from insect and acarina attack and infestation. Soil application of the formula I compounds is particularly effective for the control of the post-embryonic development stages of Coleoptera and Diptera. Applications, such as spray applications, of compositions of the invention are generally effective at rates which provide about 0.125 kg/ha to about 250 kg/ha, preferably about 10 kg/ha to 100 kg/ha. Of course, it is contemplated that higher or lower rates of application of the N-arylhydrazine derivatives may be used dependent upon the prevailing environmental circumstances such as population density, degree of infestation, stage of plant growth, soil conditions, weather conditions and the like.

Advantageously, the formula I compounds may be used in conjunction with, or in combination with other biological and chemical control agents including other insecticides, nematicides, acaricides, molluscicides, fungicides and bactericides such as nuclear polyhedrosis viruses, pyrroles, arylpyrroles, halobenzoylureas, pyrethroids, carbamates, phosphates, and the like.

Typical formulations suitable for the formula I N-arylhydrazine derivatives are granular compositions, flowable compositions, wettable powders, dusts, microemulsions, emulsifiable concentrates and the like. All compositions which lend themselves to soil, water and foliage application and provide effective plant protection are suitable. Compositions of the invention include the formula I N-arylhydrazine derivatives admixed with an inert solid or liquid carrier.

Where compositions of the invention are to be employed in combination treatments with other biological or chemical agents, the composition may be applied as an admixture of the components or may be applied sequentially.

For a more clear understanding of the invention, specific examples thereof are set forth below. These examples are merely illustrative, and are not to be understood as limiting the scope and underlying principles of the invention in any way.

### EXAMPLE 1

### Preparation of 2,2-Dimethylpropionic acid,2-(2,6-dichloro-α,α,α-trifluoro-p-tolyl)hydrazid

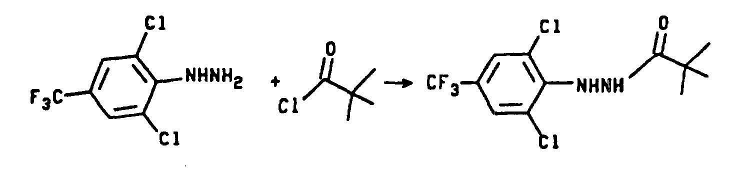

A solution of 2,6-dichloro-4-(trifluoromethyl)phenylhydrazine (50.0 g, 0.20 mol) in methylene chloride is treated dropwise with trimethylacetyl chloride (30.6 g, 0.254 mol), stirred for 30 minutes, treated with 10% aqueous NaOH and stirred for 3 hours. The phases are separated; the organic phase is washed with water, dried over MgSO₄ and concentrated in vacuo to give an off-white solid residue. The solid is recrystallized from 1,2-dichloroethane to give the title product as a white solid, 55 g (82% yield), mp 140-141°, identified by ¹HNMR, ¹³CNMR and IR spectral analyses.

### EXAMPLES 2-42

### Preparation of substituted N-arylhydrazide derivatives

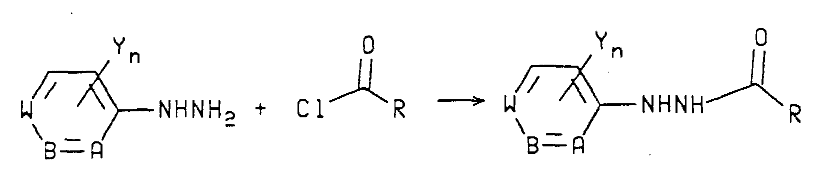

Using essentially the same procedure described above for Example 1 and substituting the appropriate arylhydrazine and acid chloride, the compounds shown in Table I are prepared and identified by ¹HNMR, ¹³NMR and IR spectral analyses.

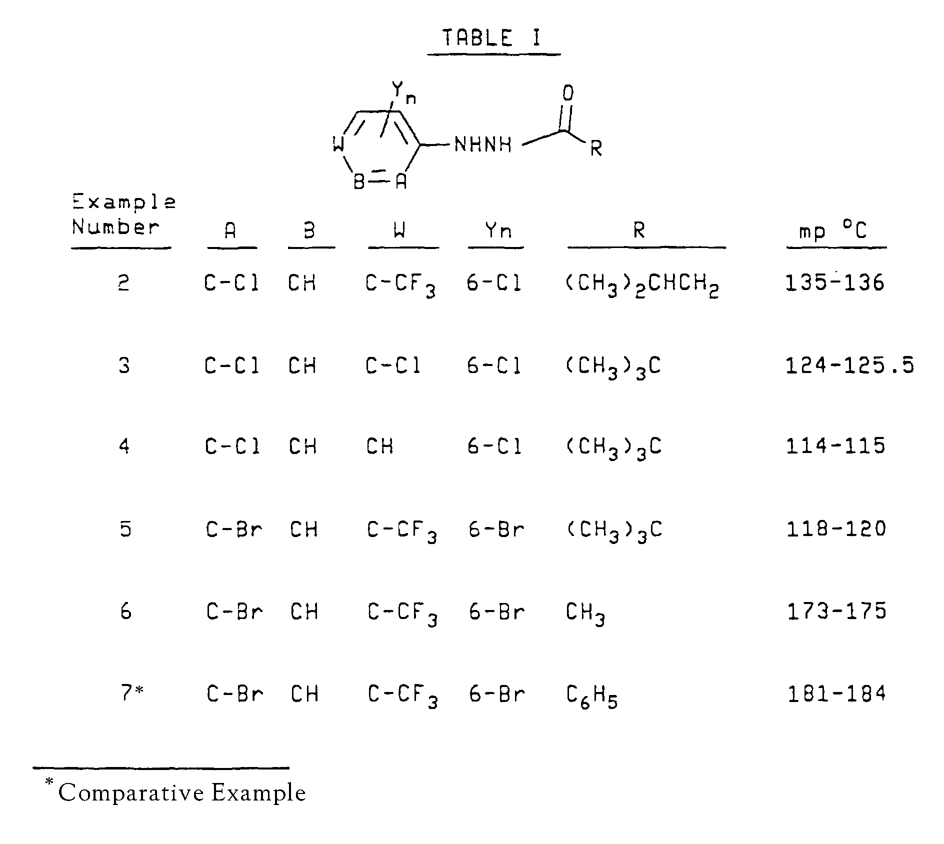

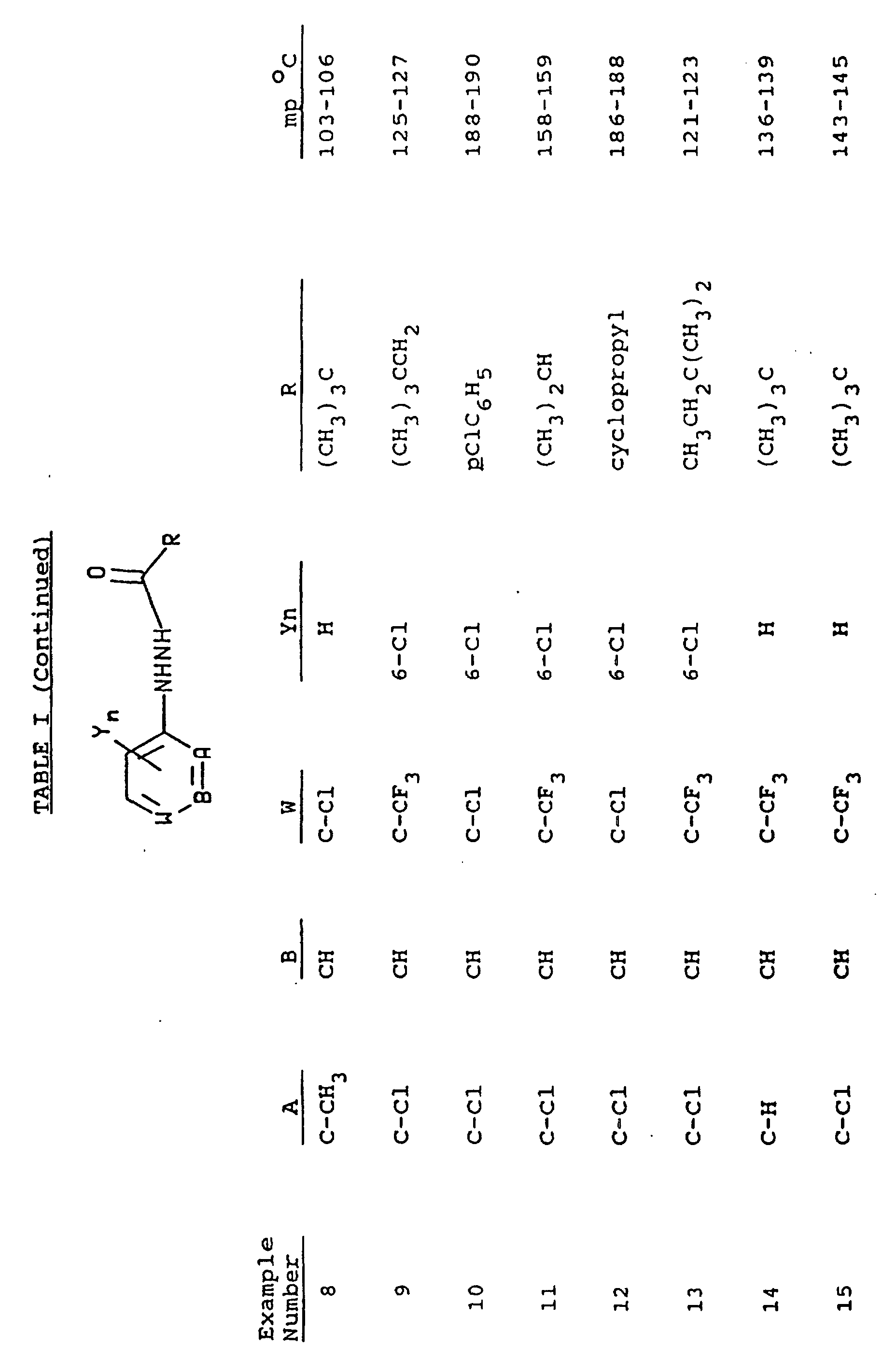

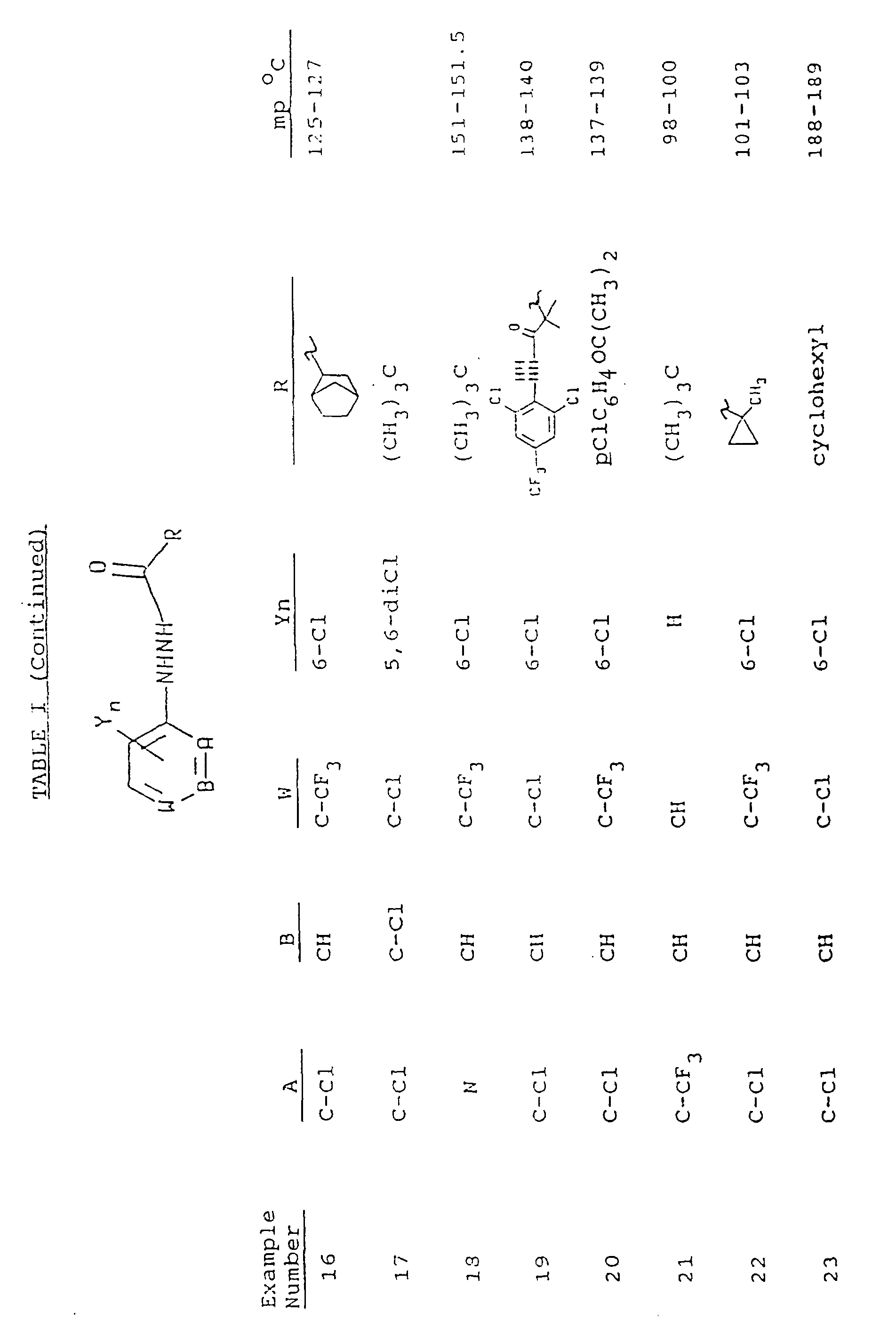

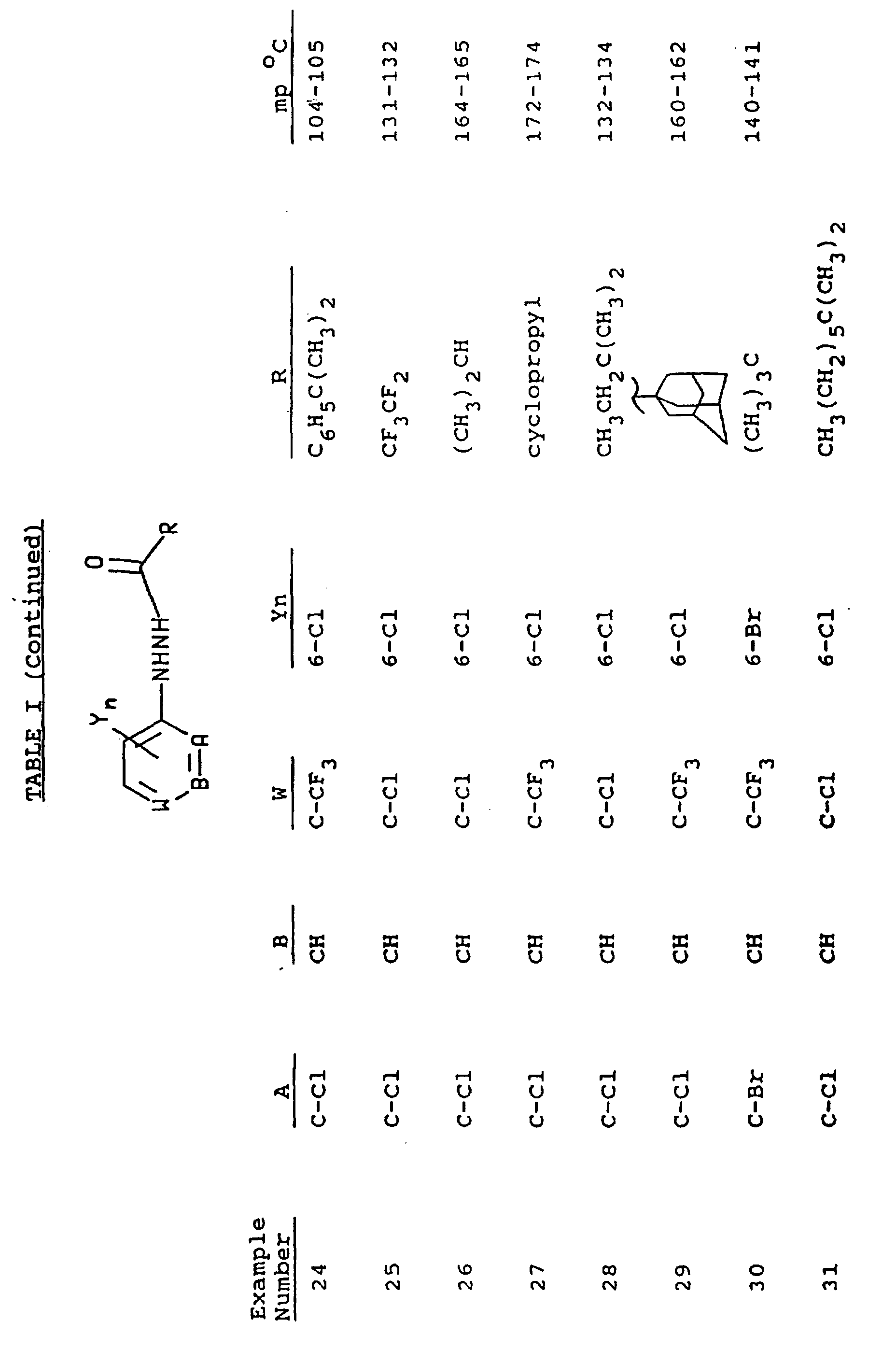

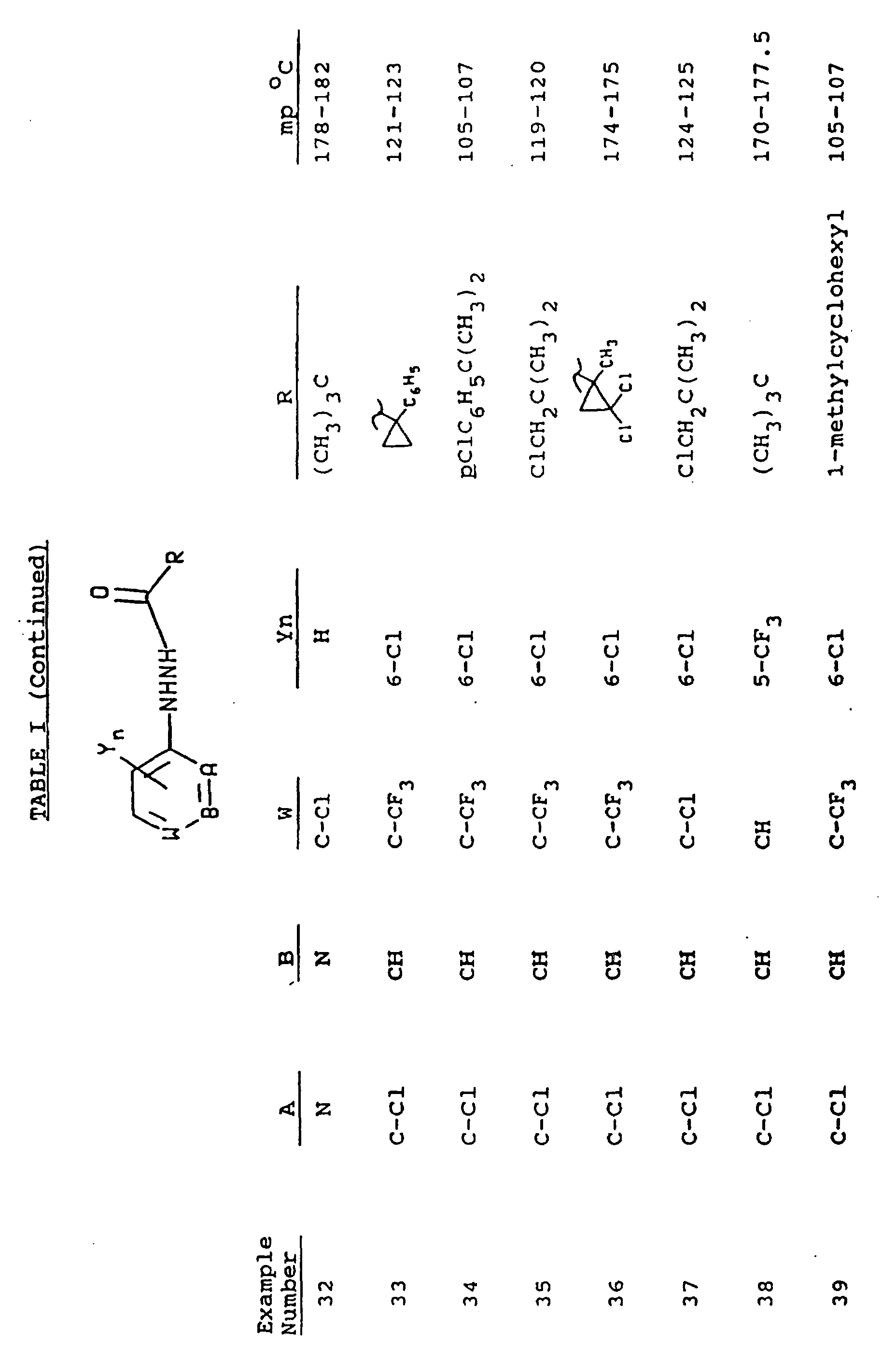

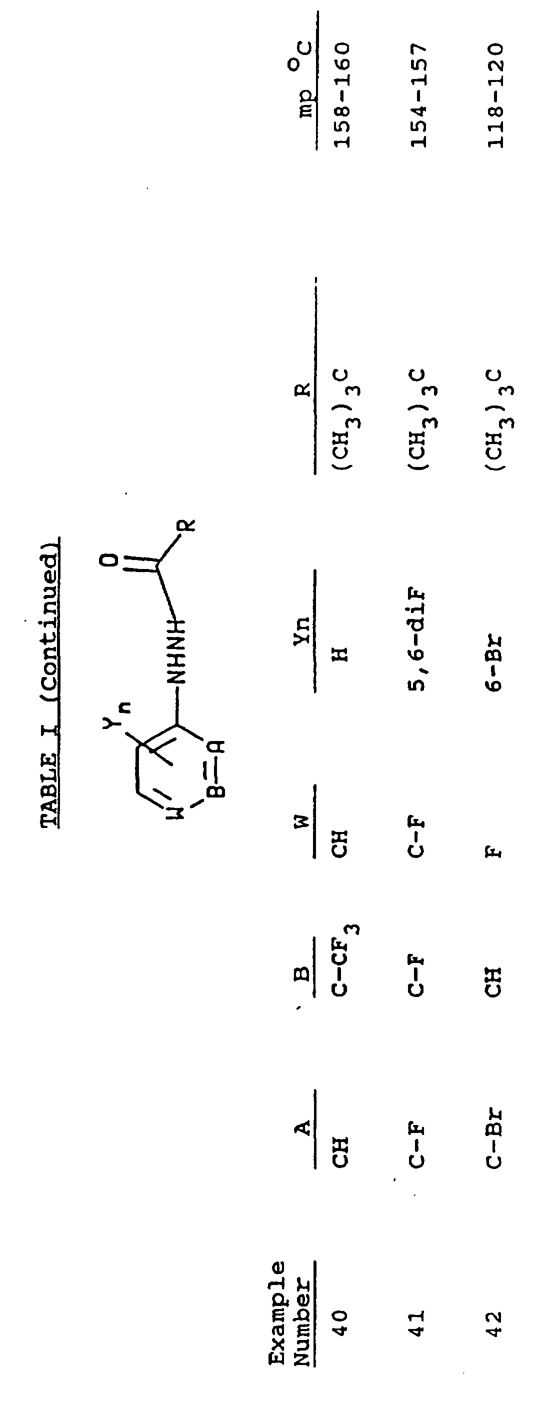

### COMPARATIVE EXAMPLE 43

### Preparation of 1-chloro-2,2-dimethylpropionaldehyde, 2-(2,6-Dichloro-α,α,α-trifluoro-p-tolyl)hydrazone

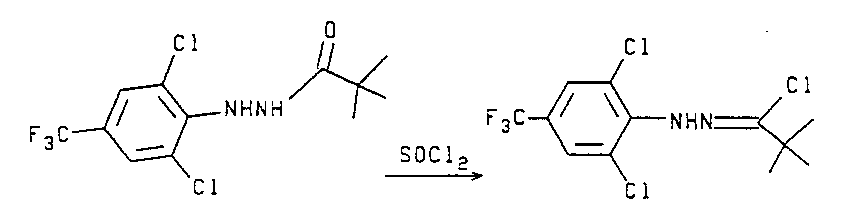

A mixture of 2,2-dimethyl-2-(2,6-dichloro-α,α,α-trifluoro-D-tolyl)hydrazide propionic acid (50.0 g, 0.152 mol) and thionyl chloride (53.8 g, 0.452 mol) in toluene is heated at reflux temperature for 8 hours, cooled to room temperature and concentrated in vacuo to give an oil residue. The oil is dissolved in hexanes and passed through a silica gel filtercake. The filtercake is washed with several portions of hexanes. The filtrates are combined and concentrated in vacuo to give the title product as a yellow oil, 47.2 g (90% yield), identified by ¹HNMR, ¹³CNMR and IR spectral analyses.

### COMPARATIVE EXAMPLES 44-84

### Preparation of substituted N-arylhydrazinoyl chlorides

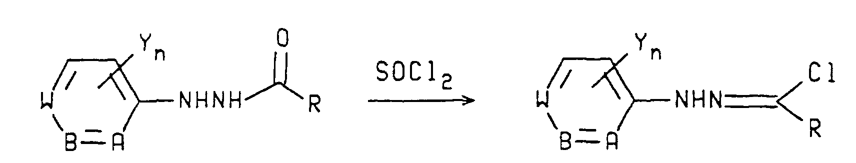

Using essentially the same procedure as described above in Example 43 and substituting the appropriate hydrazide substrate, the compounds shown in Table II are prepared and identified by ¹HMR, ¹³CNMR and IR spectral analyses.

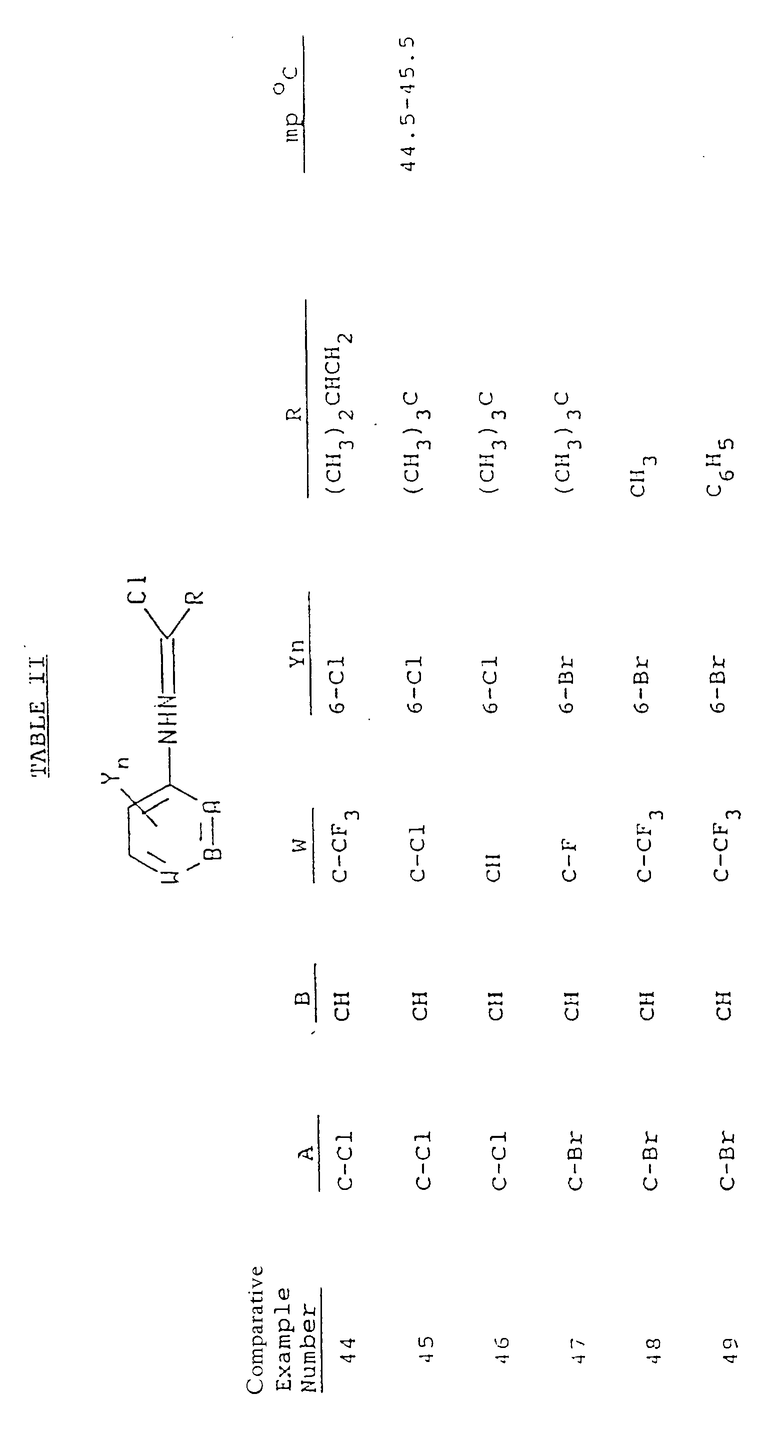

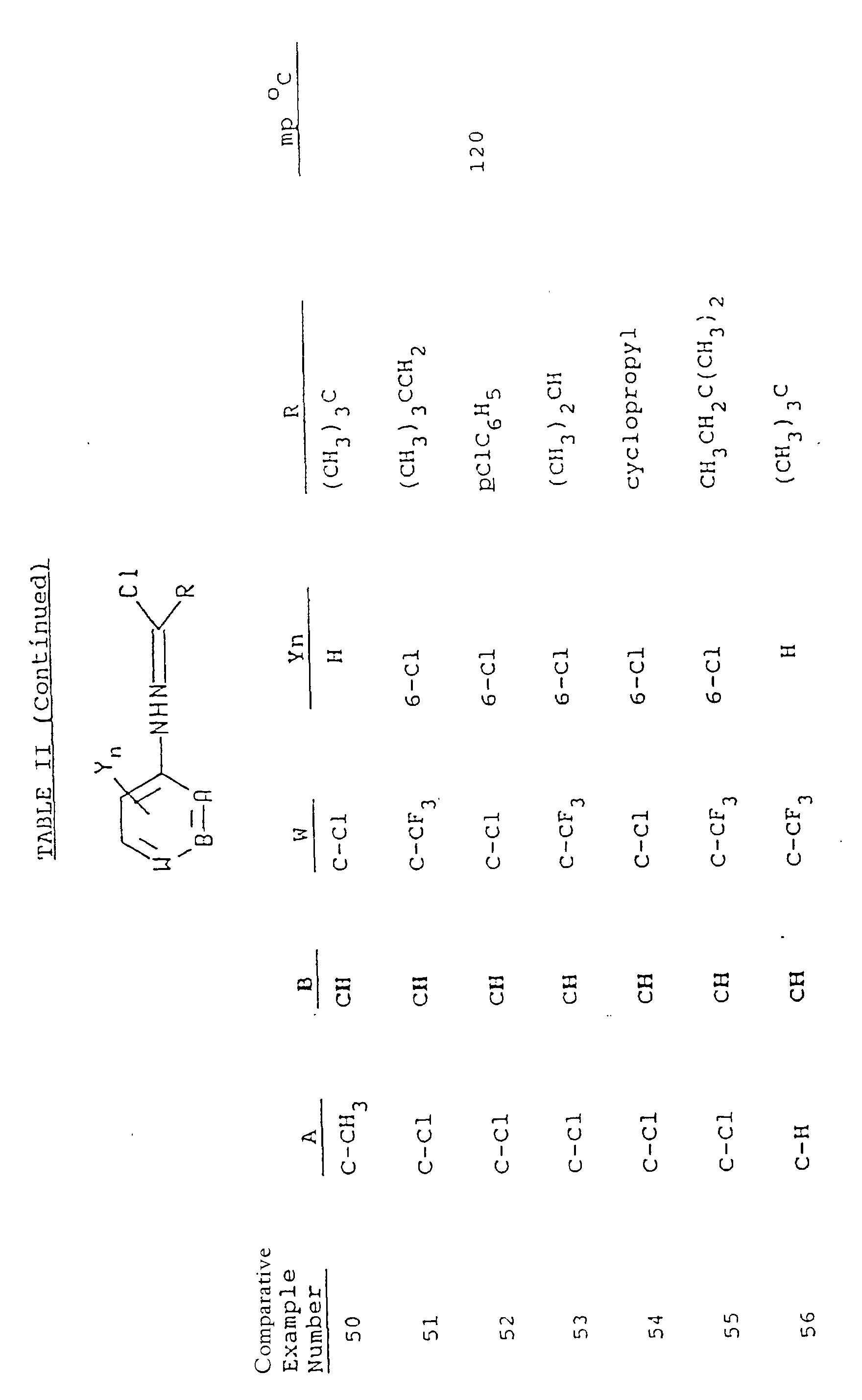

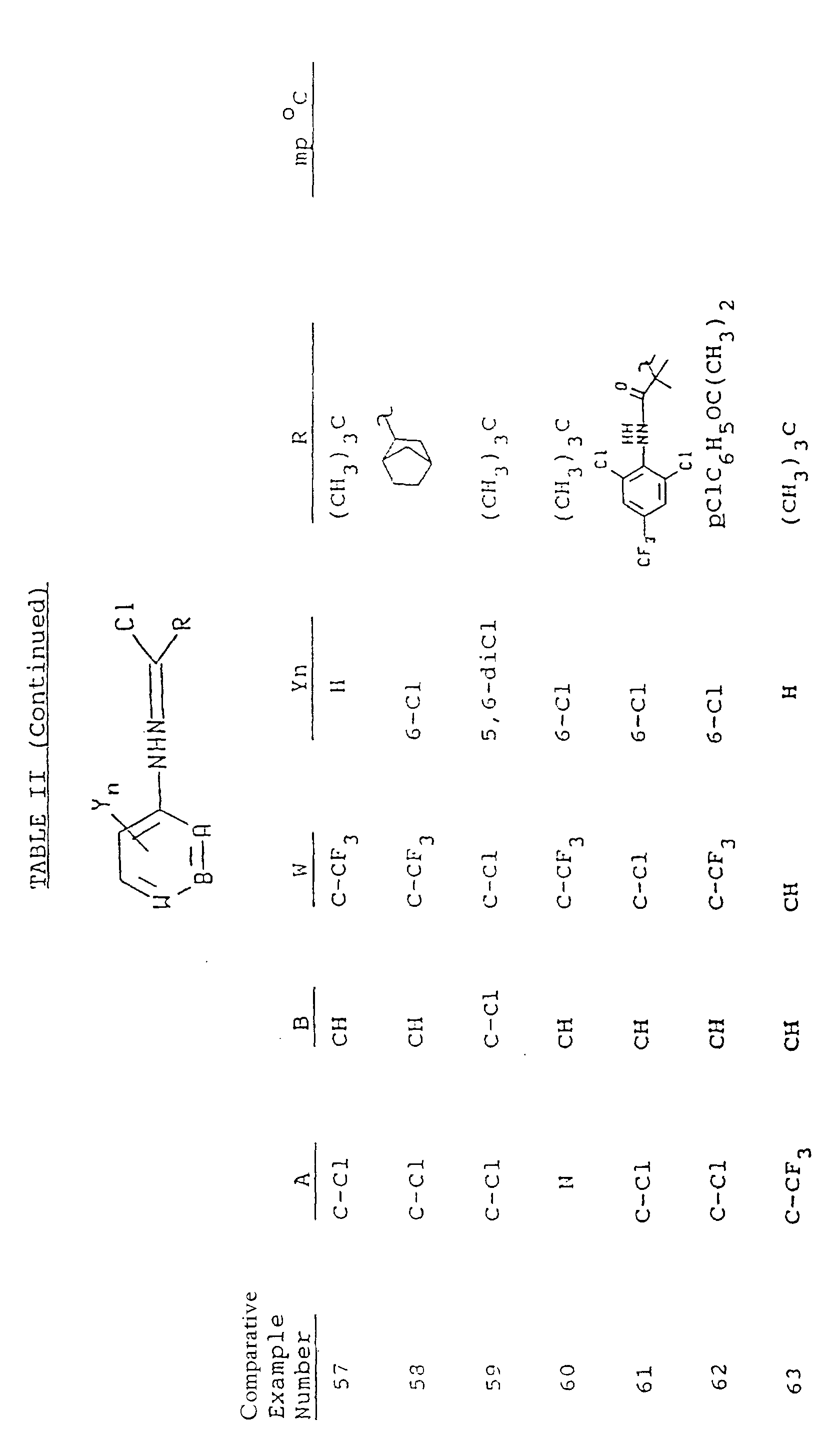

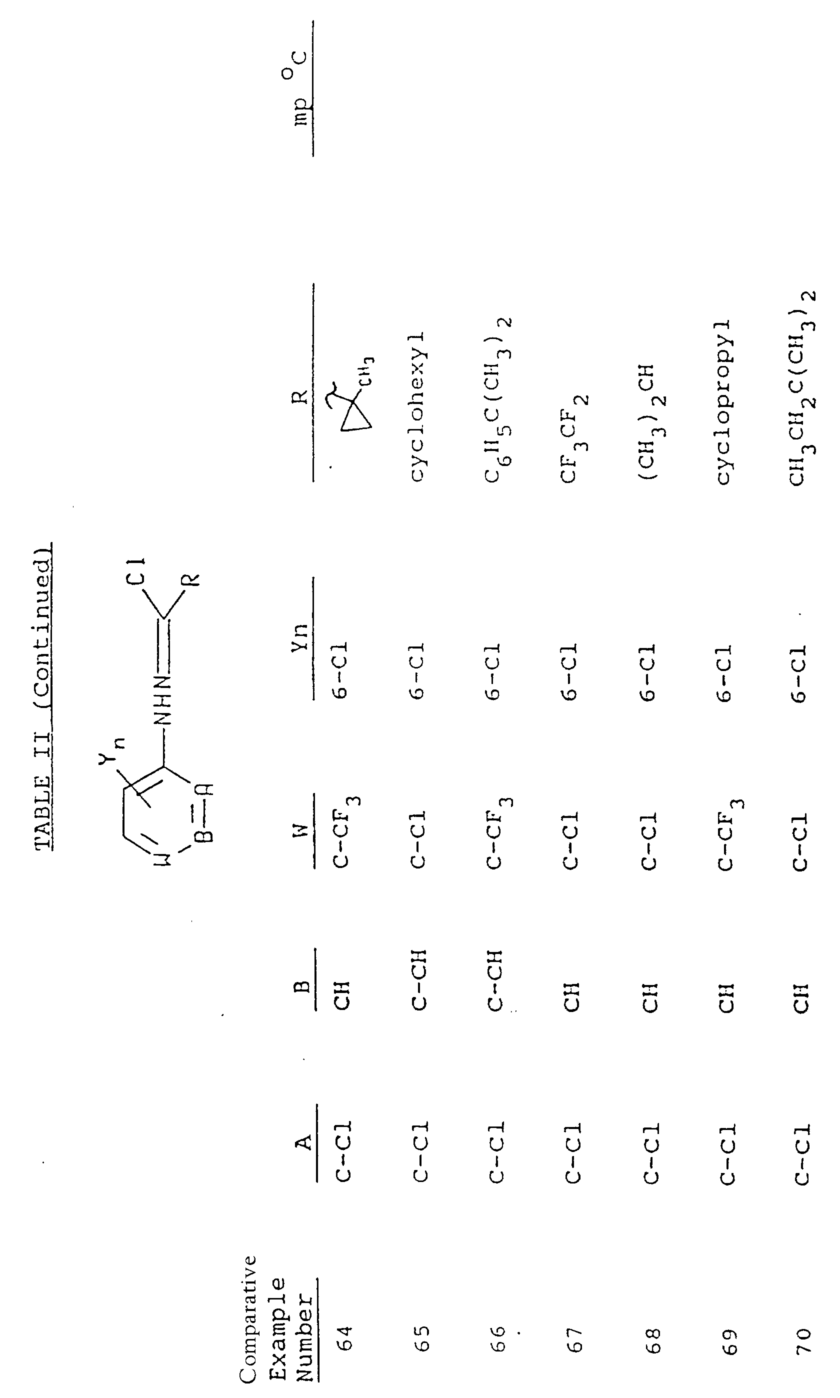

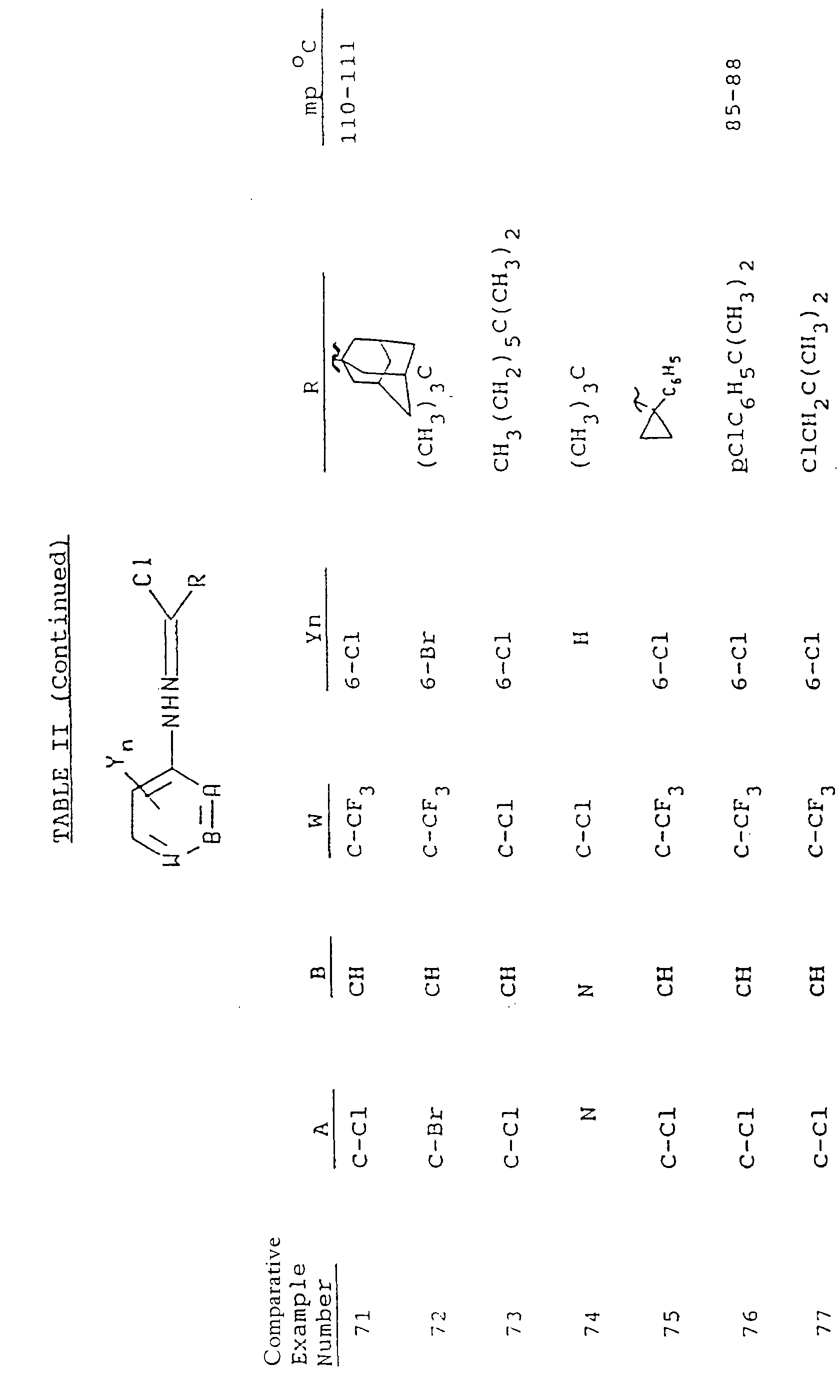

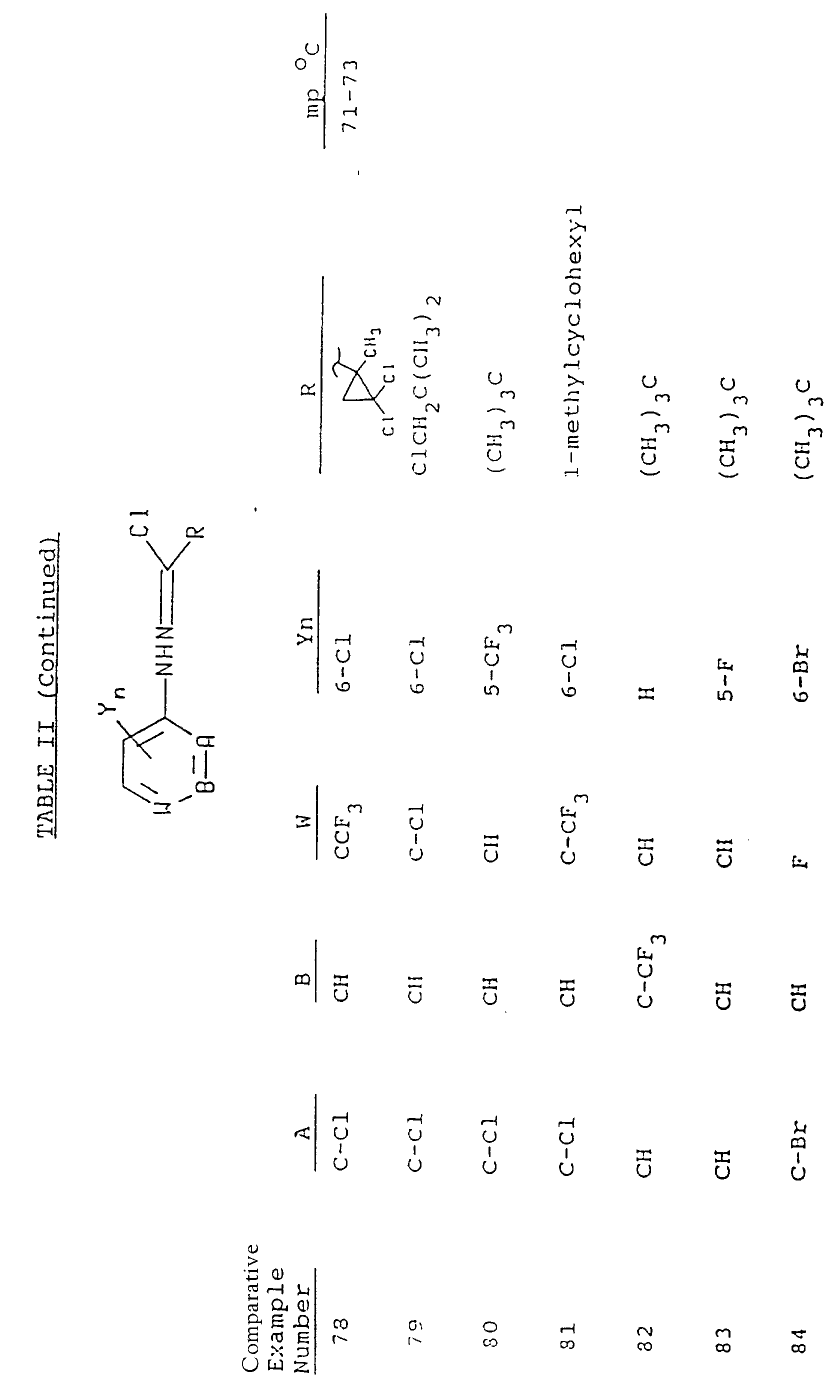

### EXAMPLE 85

### Preparation of N-Ethyl-2,2-dimethylpropionamide, 2-(2,6-Dichloro-α,α,α-trifluoro-p-tolylhydrazone

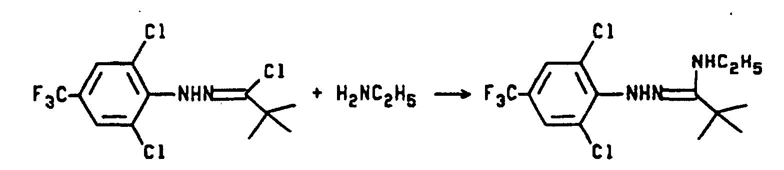

A solution of (2,6-dichloro-α,α,α-trifluoro-p-tolyl)hydrazone 1-chloro-2,2-dimethylpropionaldehyde (20.0 g, 0.0575 mol) in tetrahydrofuran is treated dropwise with 70% aqueous ethylamine (28.0 g, 0.144 mol) at room temperature, stirred for 1 hour and concentrated in vacuo to give a semi-solid residue. The semi-solid is dispersed in ether and water. The phases are separated; the organic phase is washed with water, dried over MgSO₄ and concentrated in vacuo to give the title product as a yellow oil, 19.8 g (97% yield), identified by ¹HNMR, ¹³CNMR and IR spectral analyses.

### EXAMPLES 86-169

### Preparation of substituted N-arylamidrazones

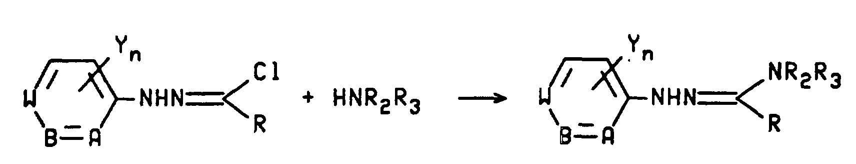

Using essentially the same procedure described above in Example 85 and substituting the appropriate hydrazinoylchloride and a suitable amine, the compounds shown in Table III are prepared and identified by ¹HNMR, ¹³CNMR and IR spectral analyses.

Hydrochloride salts of the invention may be prepared in accordance with the procedure outlined below.

### Example 146 - Preparation of N-Ethvl-2,2-dimethyl-proprionamide,2-(2,6-dichloro-α,α,α-trifluoro-p-tolylhydrazone hydrochloride

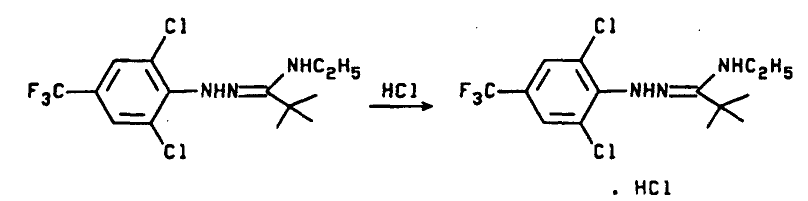

A stirred mixture of N-ethyl-2,2-dimethylpropionamide, 2-(2,6-dichloro-α,α,α-trifluoro-p-tolylhydrazone (0.1 g, 2.8 mmol) and hexane is bubbled through with HCl gas for a 30 minute period. The resultant reaction mixture is filtered to give the title compound as a white solid, 1.13 g, mp 202-202.5°C.

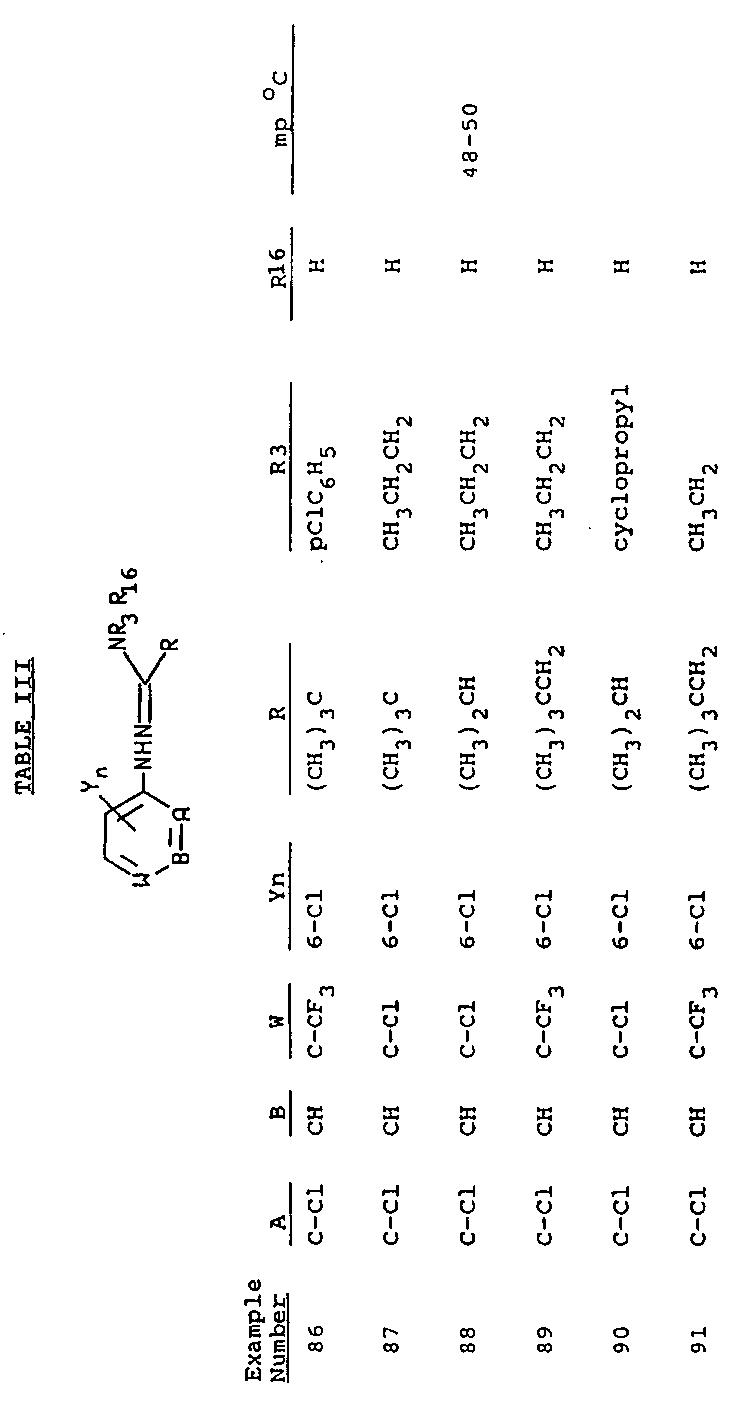

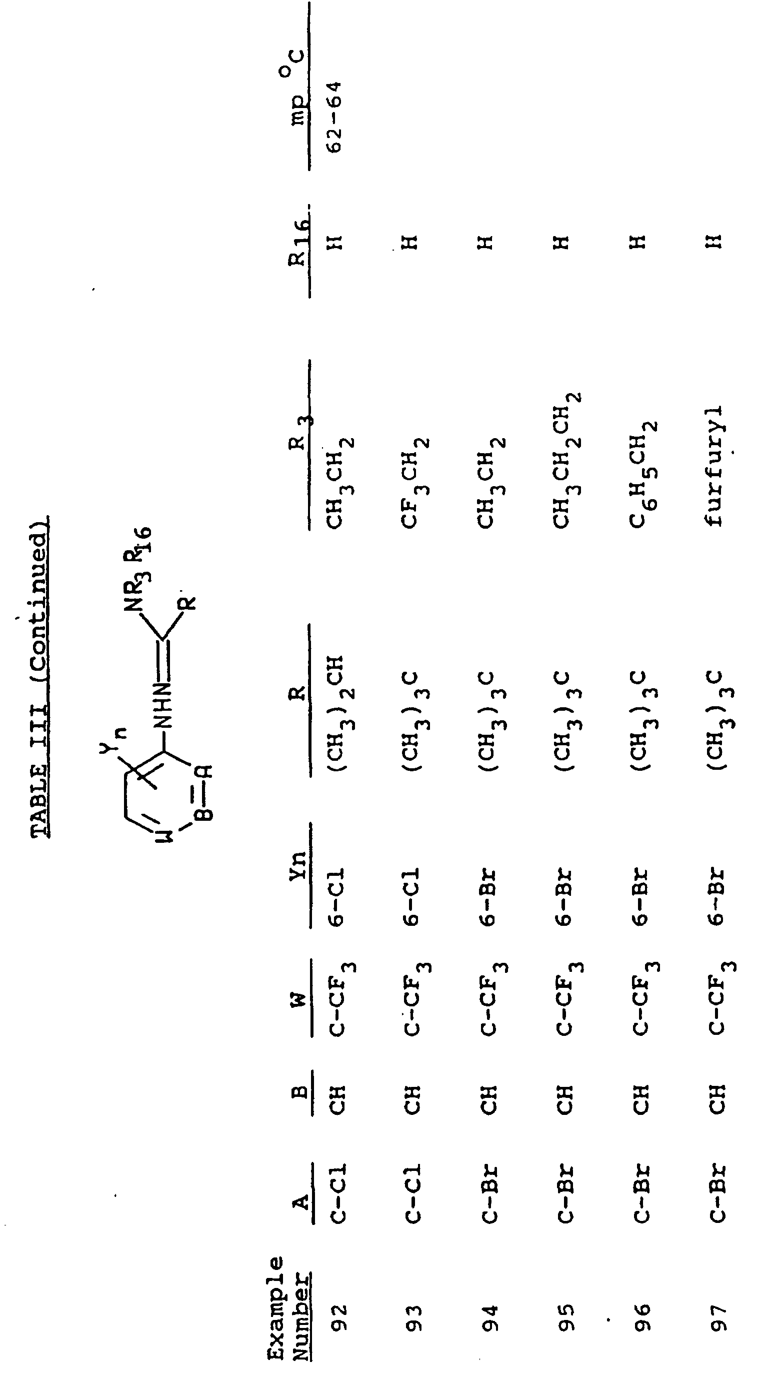

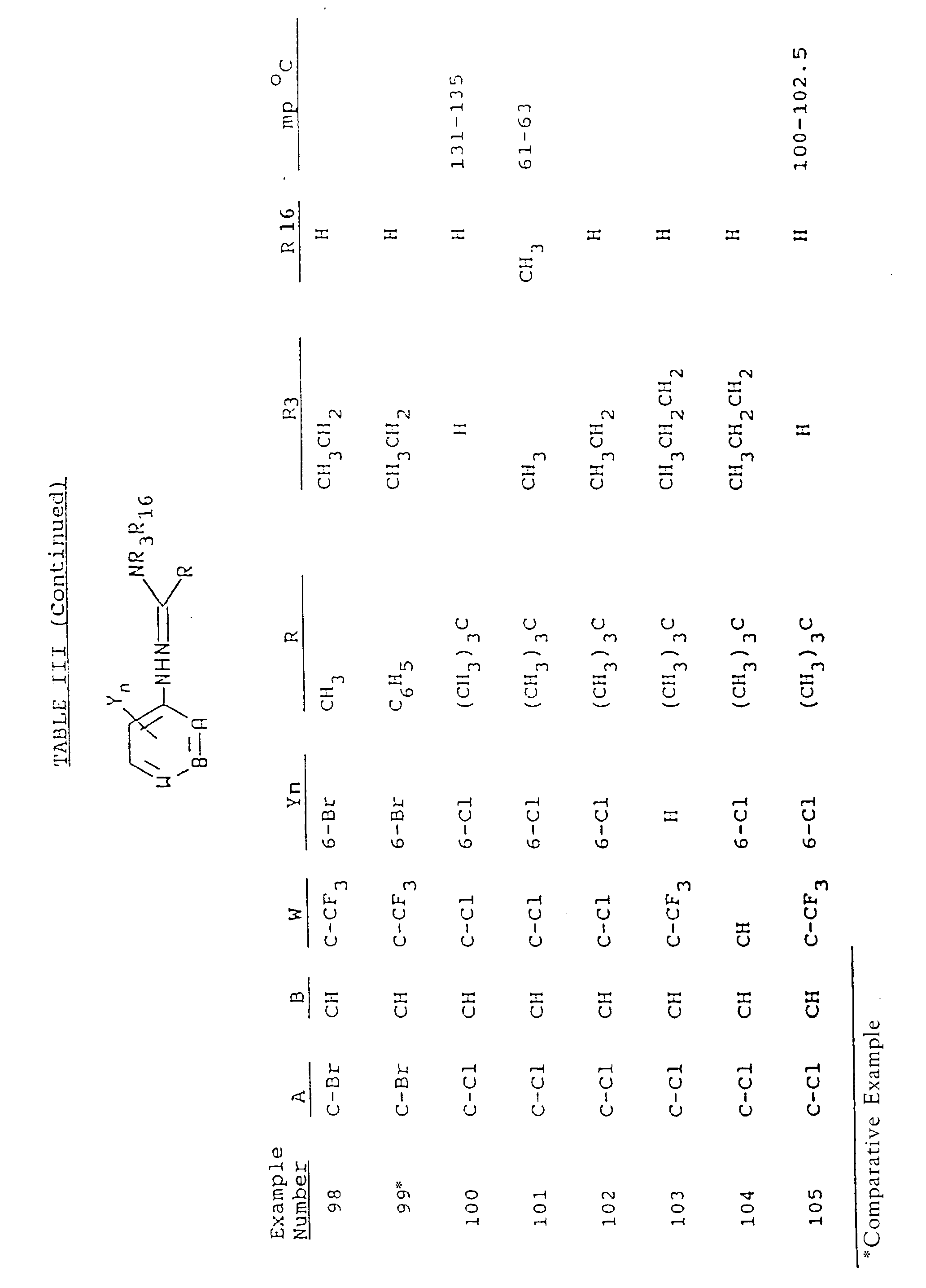

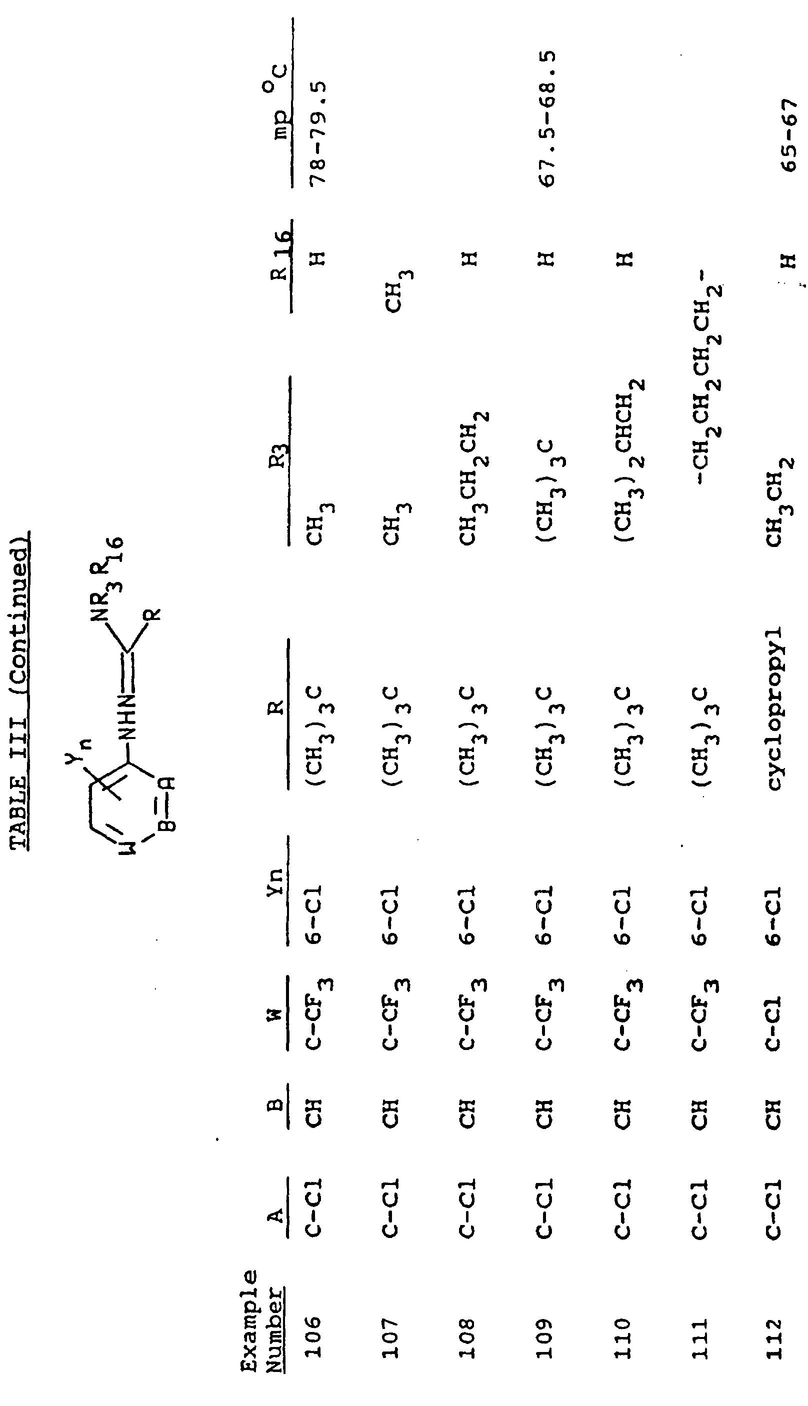

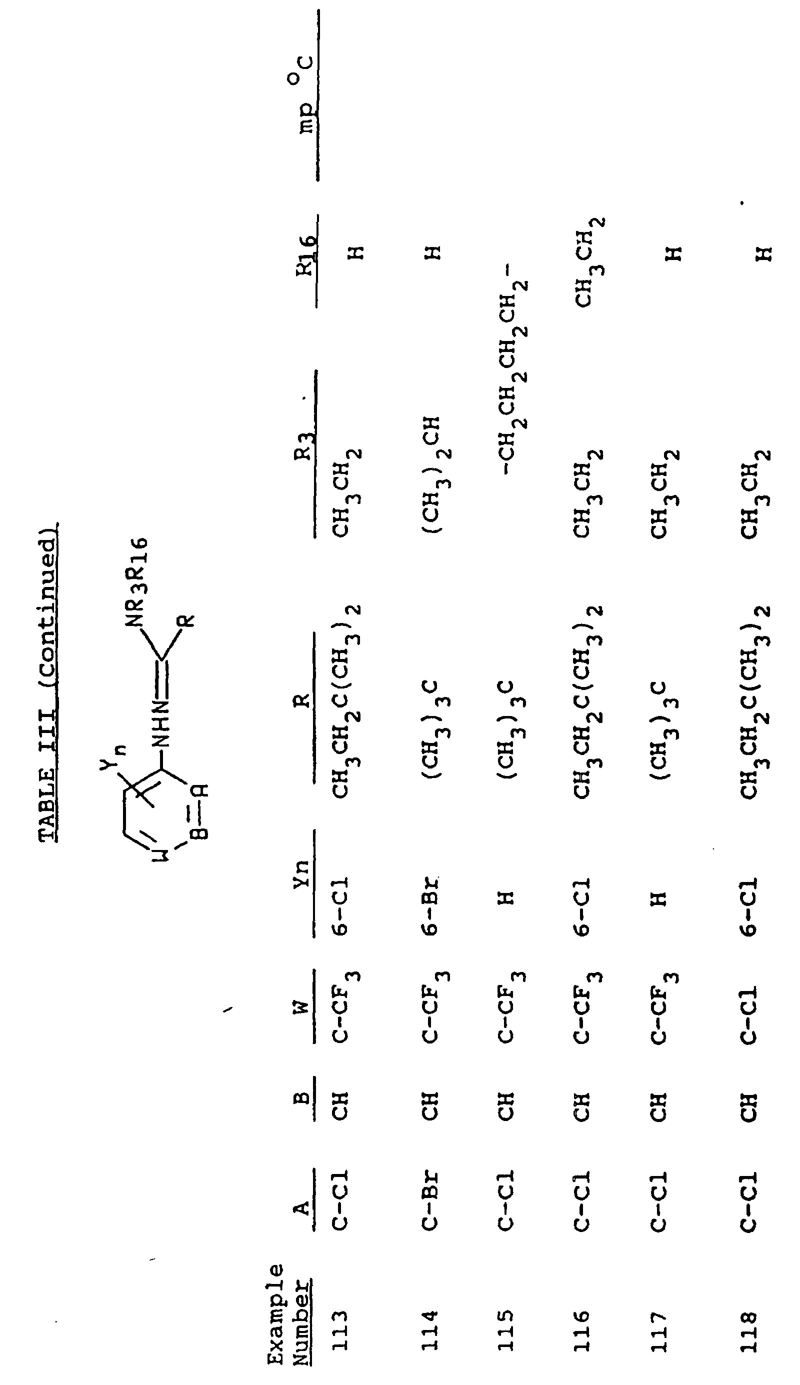

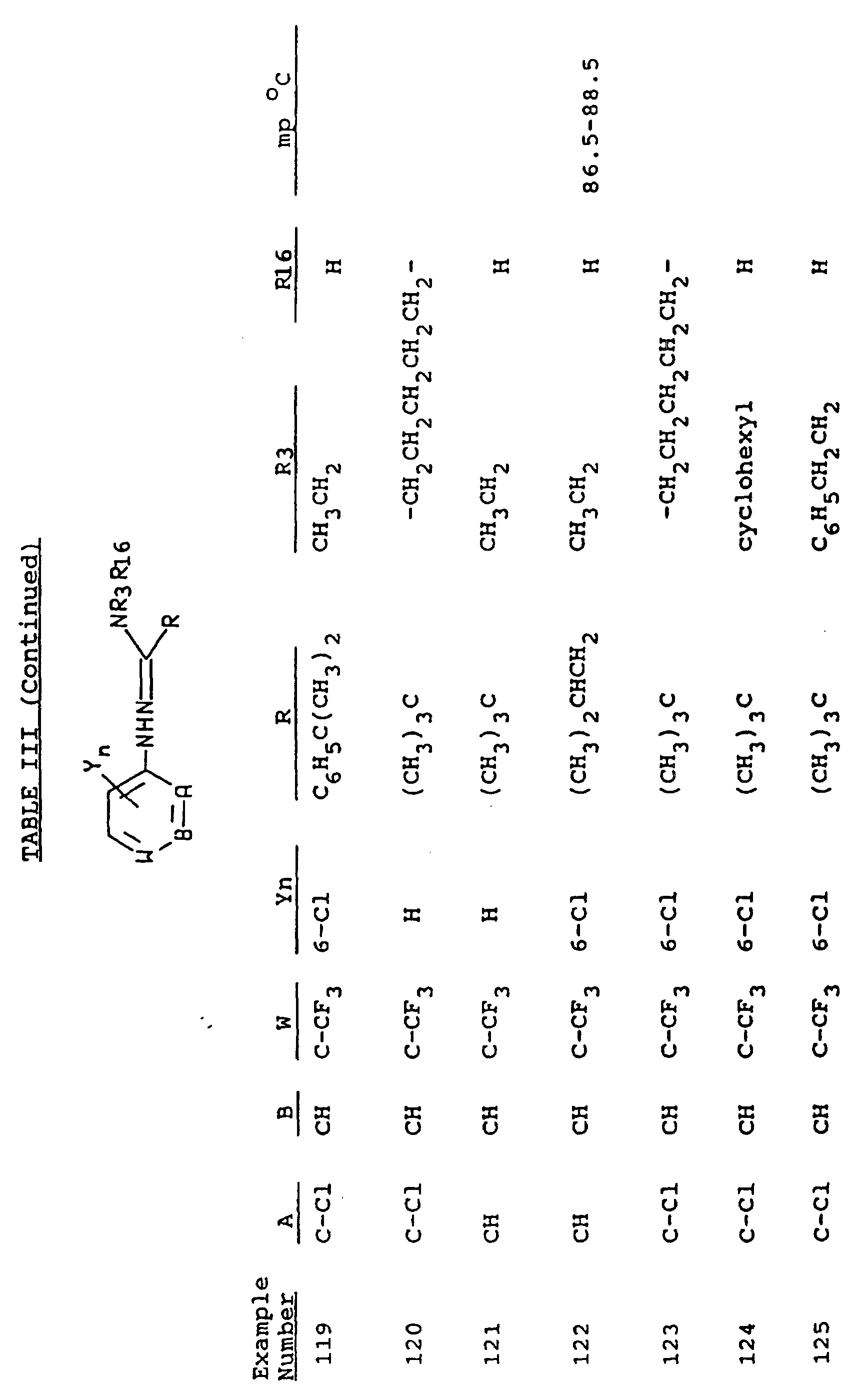

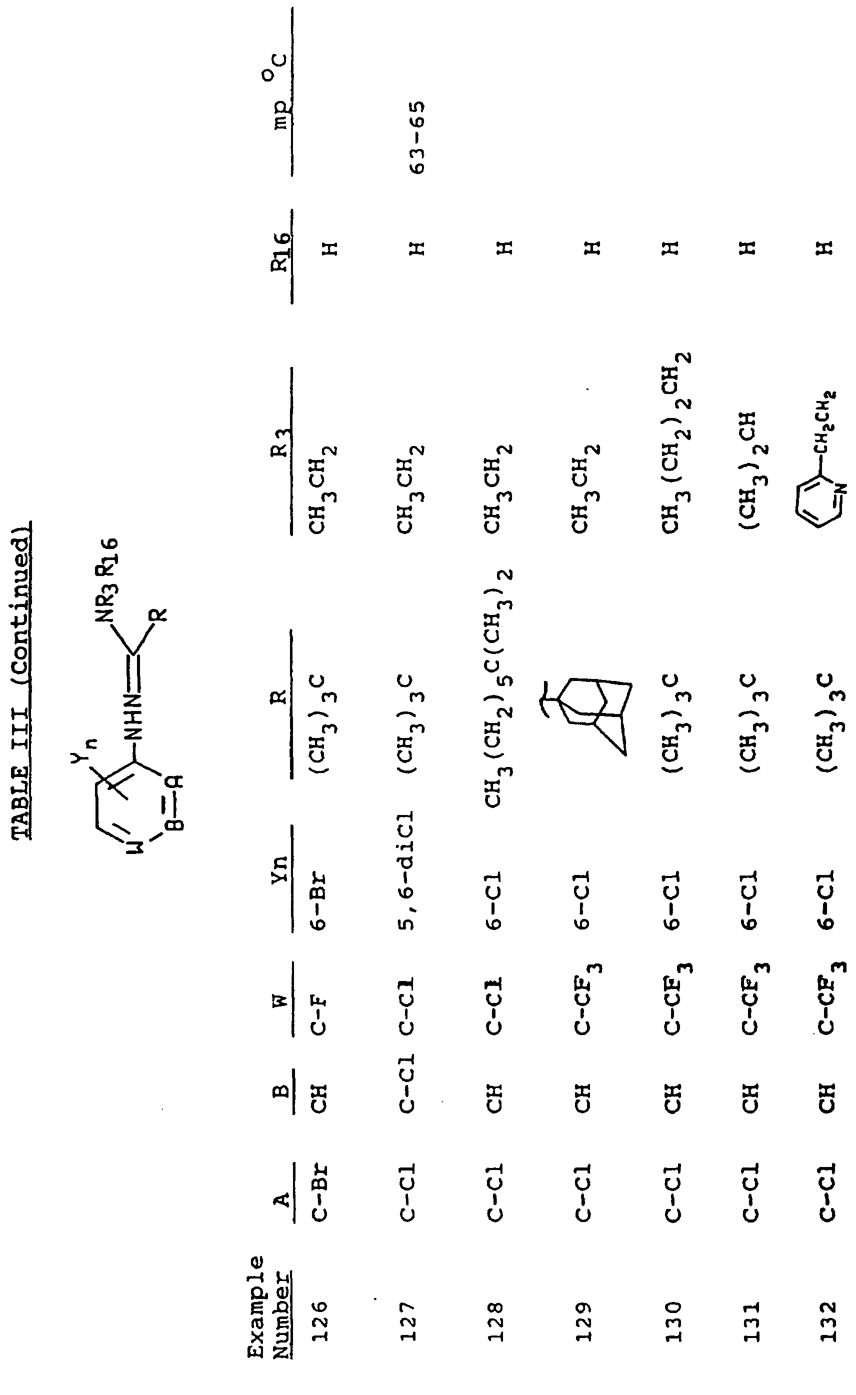

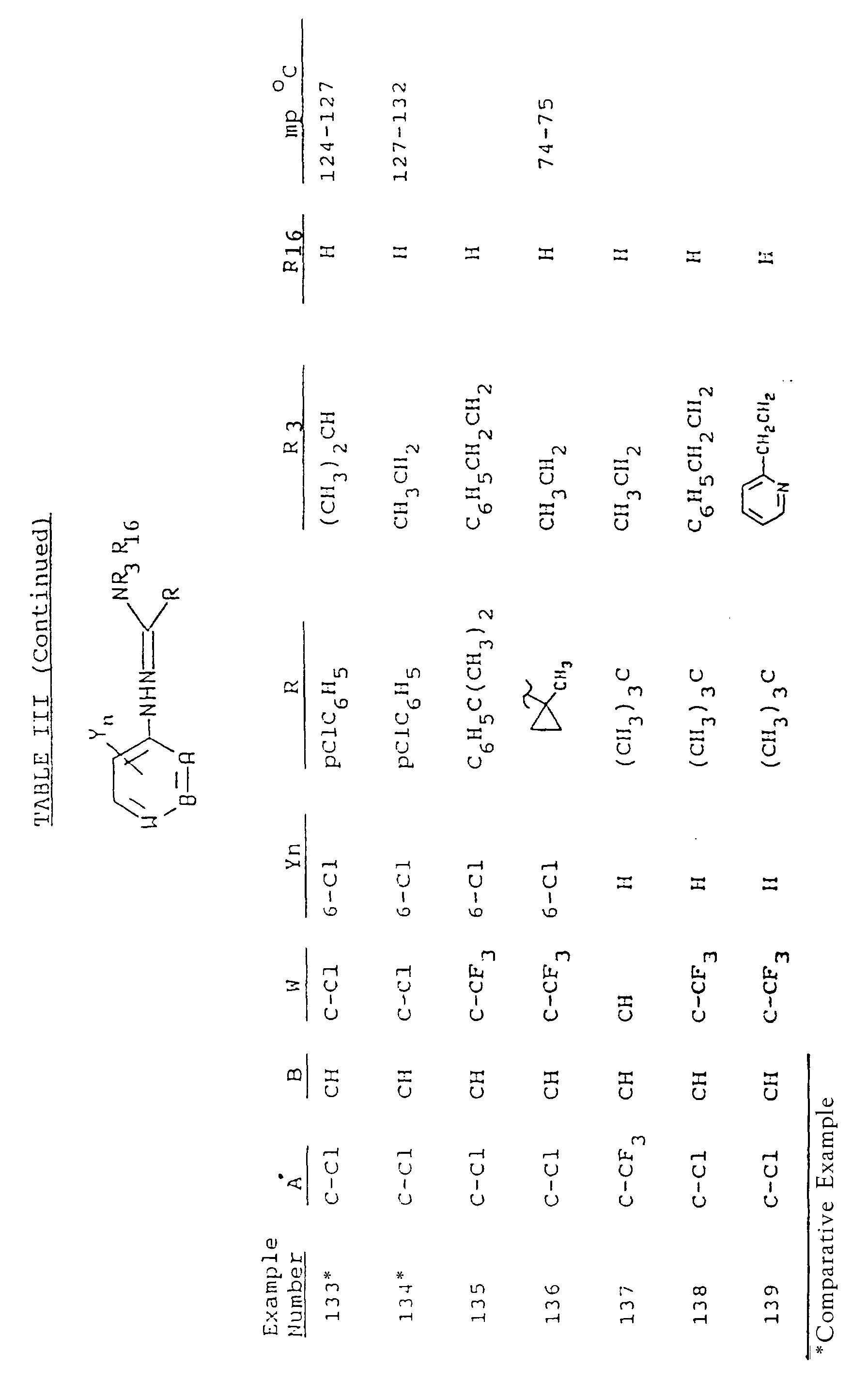

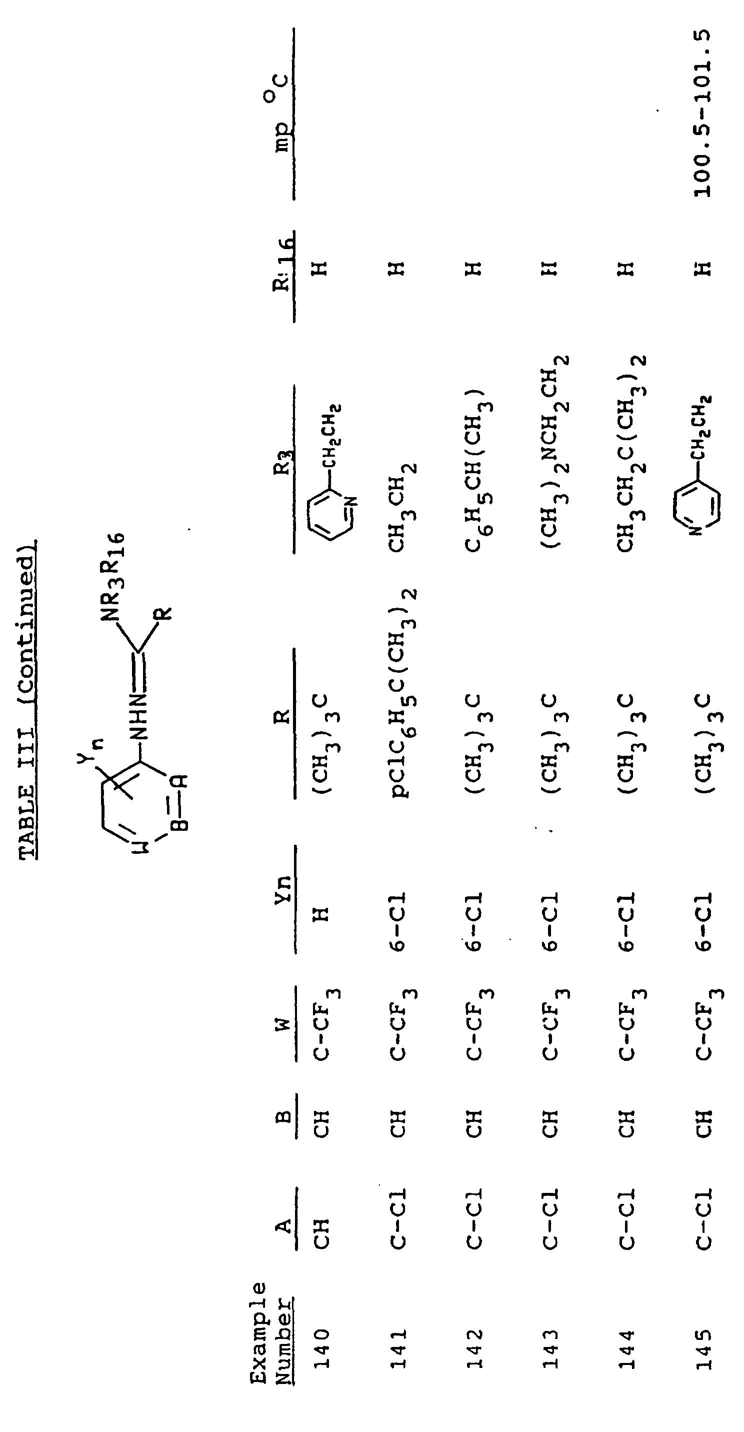

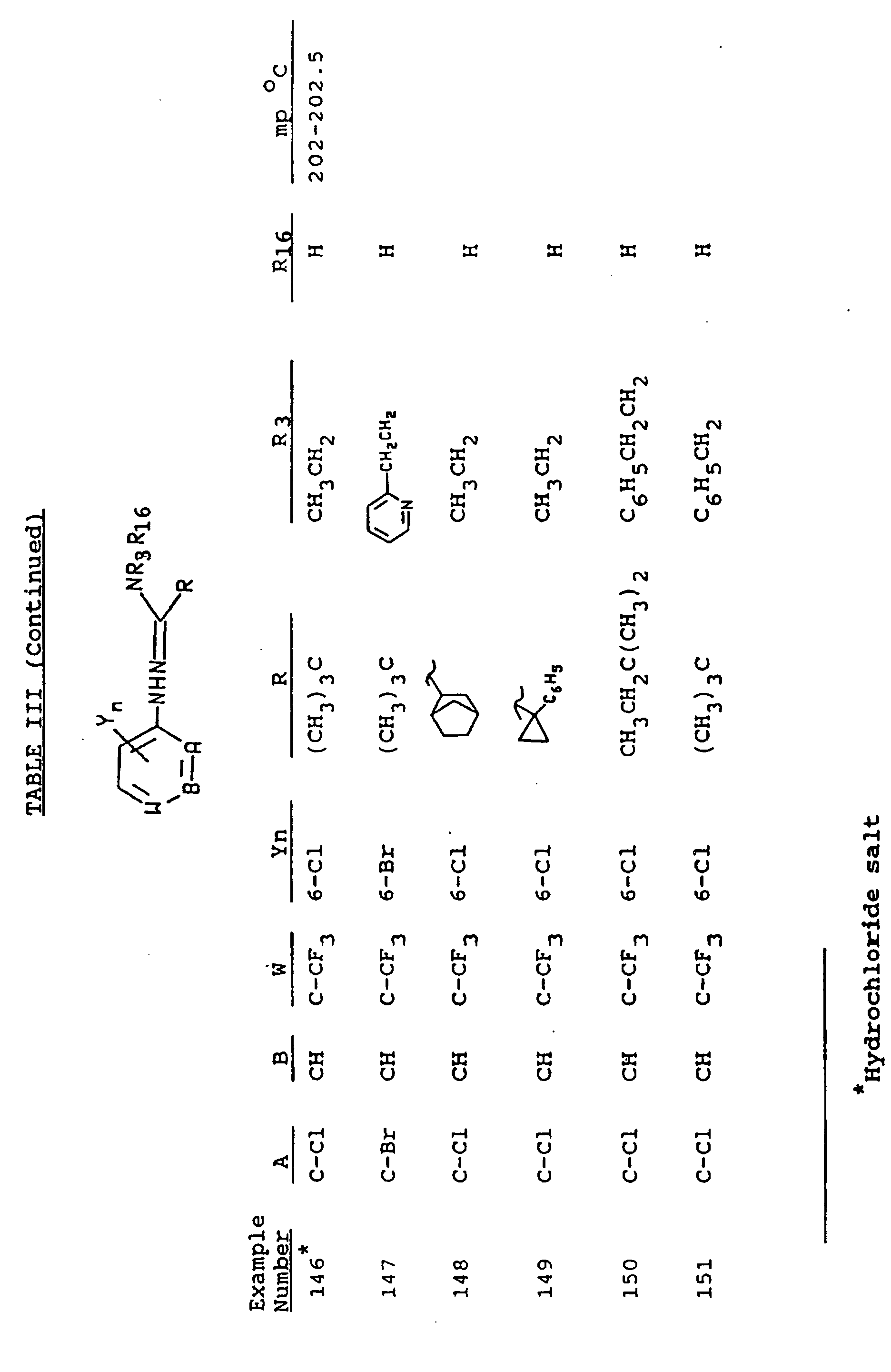

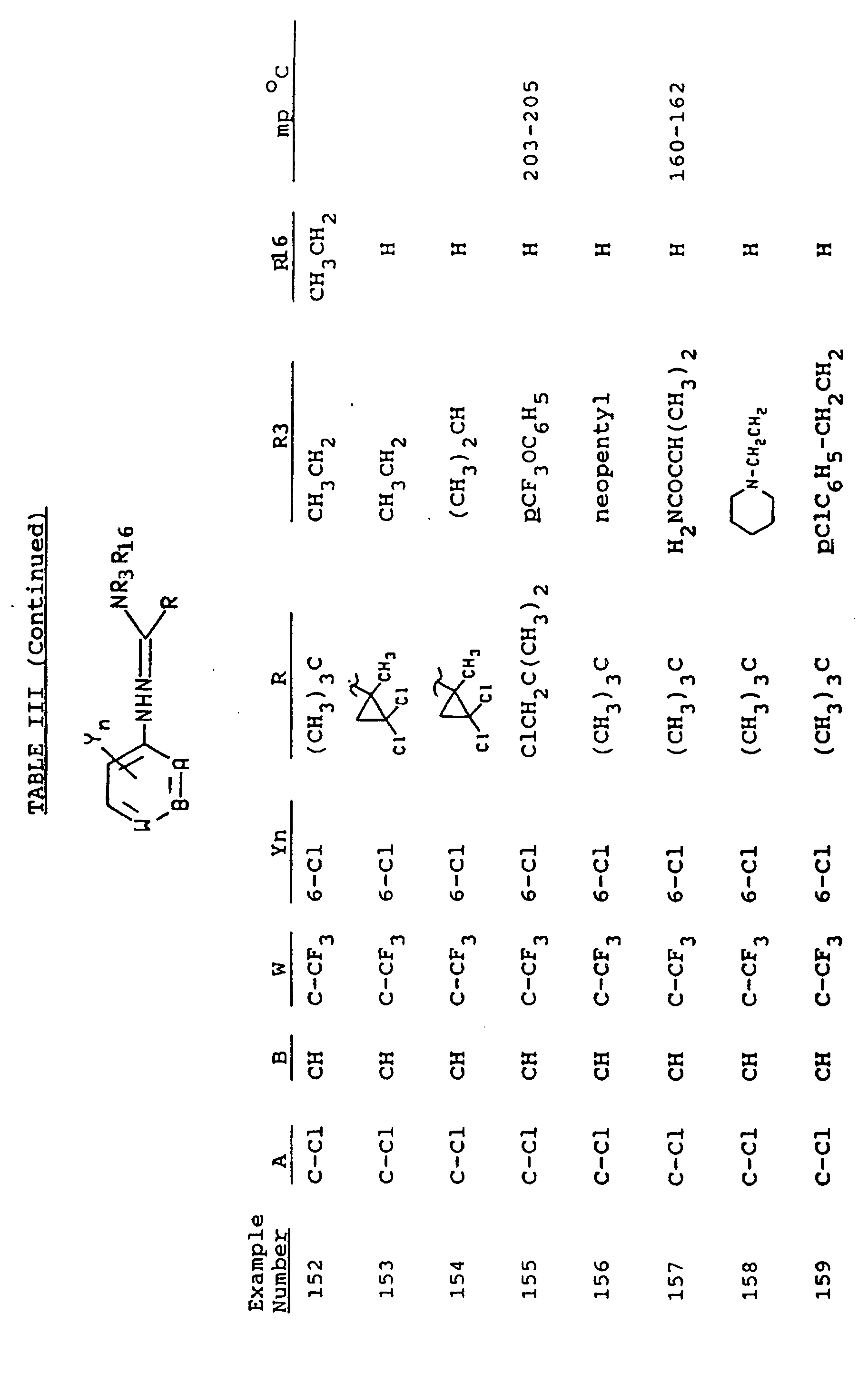

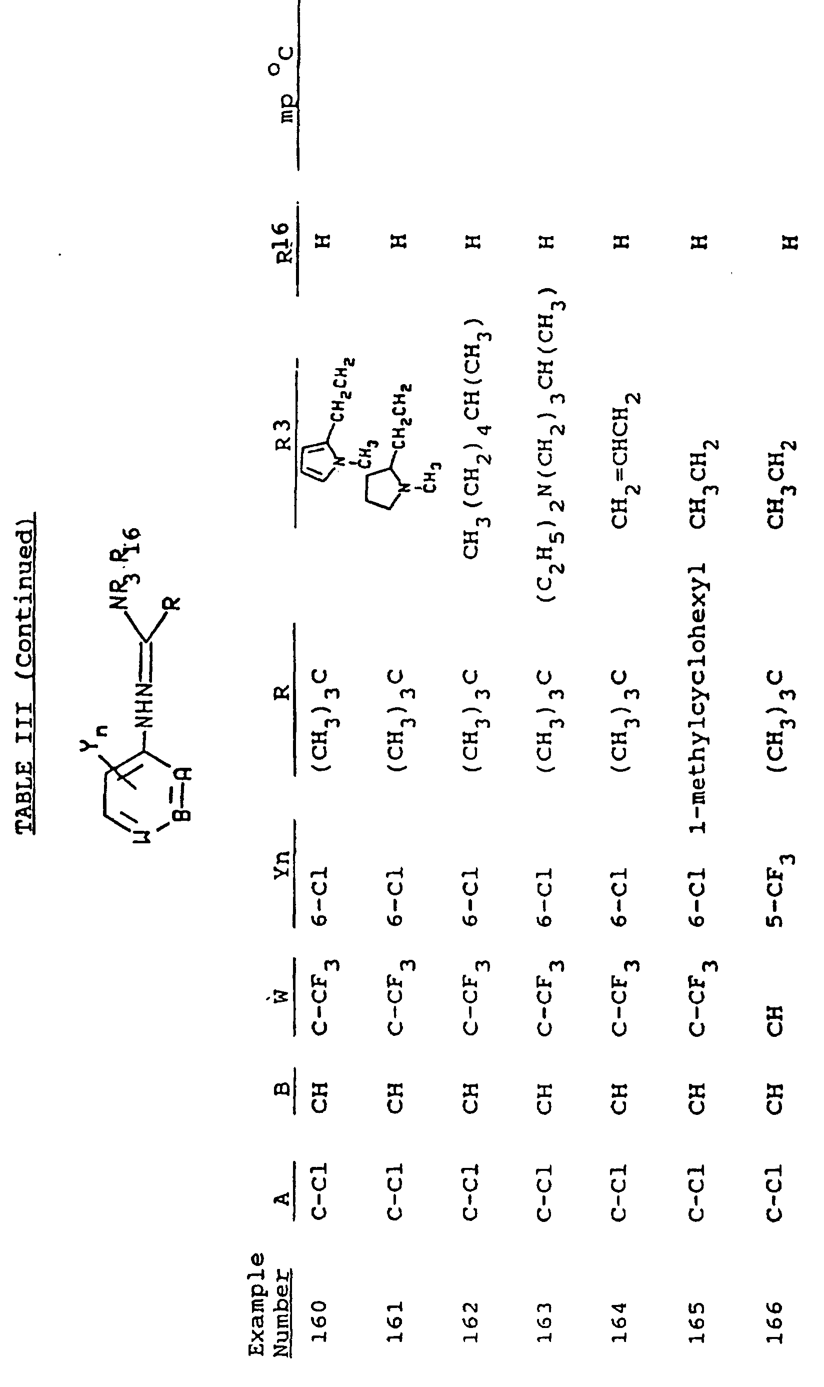

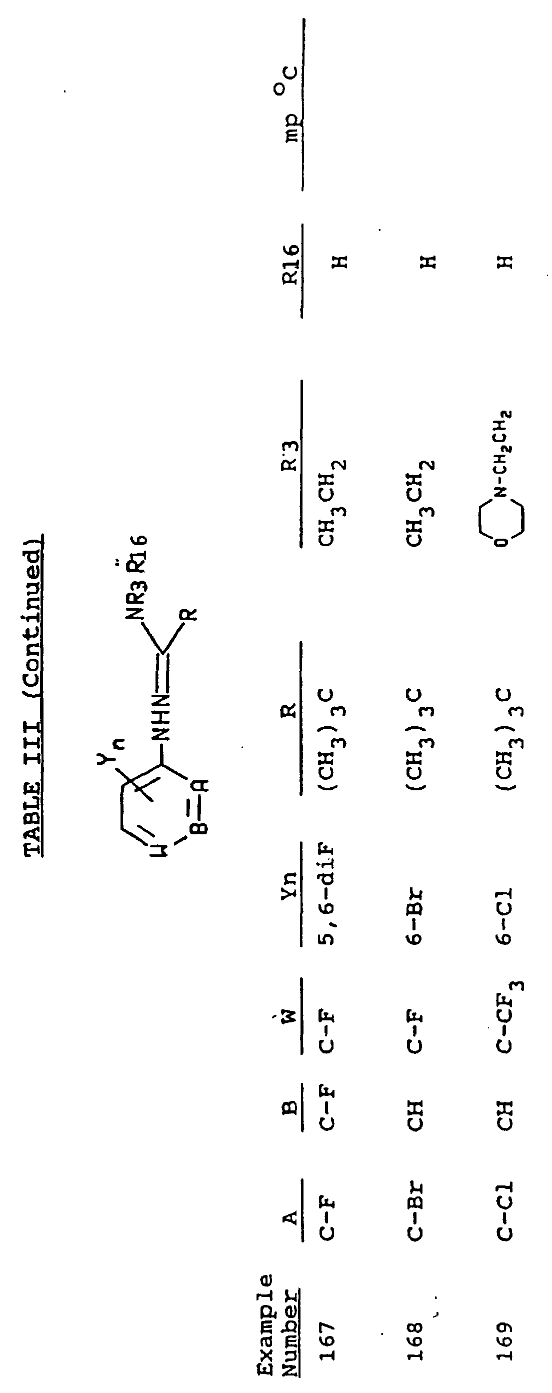

### EXAMPLE 170

### Insecticidal and Acaricidal Evaluation of N-arylhydrazine Derivatives

Test solutions are prepared by dissolving the test compound in a 35% acetone in water mixture to give a concentration of 10,000 ppm. Subsequent dilutions are made with water as needed.

### Spodoptera eridania, 3rd instar larvae, southern armyworm

A Sieva limabean leaf expanded to 7-8 cm in length is dipped in the test solution with agitation for 3 seconds and allowed to dry in a hood. The leaf is then placed in a 100 x 10 mm petri dish containing a damp filterpaper on the bottom and ten 3rd instar caterpillars. At 3 and 5 days, observations are made of mortality, reduced feeding, or any interference with normal molting.

### Tetranychus urticae (OP-resistant strain), 2-spotted spider mite

Sieva limabean plants with primary leaves expanded to 7-8 cm are selected and cut back to one plant per pot. A small piece is cut from an infested leaf taken from the main colony and placed on each leaf of the test plants. This is done about 2 hours before treatment to allow the mites to move over to the test plant to lay eggs. The size of the cut, infested leaf is varied to obtain about 100 mites per leaf. At the time of test treatment, the piece of leaf used to transfer the mites is removed and discarded. The newly mite-infested plants are dipped in the test solution for 3 seconds with agitation and set in the hood to dry. After 2 days, one leaf is removed and mortality counts are made. After 5 days, another leaf is removed and observations are made of mortality of the eggs and/or newly emerged nymphs.

### Diabrotic undecimpunctata howardi, 3rd instar southern corn rootworm

One cc of fine talc is placed in a 30 ml wide-mouth screw-top glass jar. One mL of the appropriate acetone test solution is pipetted onto the talc so as to provide 1.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed on a Vortex Mixer. Following this, ten 3rd instar root-worms are added to each jar and the jars are loosely capped to allow air exchange for the larvae. The treatments are held for 6 days when mortality counts are made. Missing larvae are presumed dead, since they decompose rapidly and can not be found. The concentrations used in this test correspond approximately to 50 kg/ha.

The tests are rated according to the scale shown below and the data obtained are shown in Tables IV, V and VI.

| **RATING SCALE** | | | |
|---|---|---|---|
| Rate | % Mortality | Rate | % Mortality |
| 0 | no effect | 5 | 56-65 |
| 1 | 10-25 | 6 | 66-75 |
| 2 | 26-35 | 7 | 76-85 |
| 3 | 36-45 | 8 | 86-99 |
| 4 | 46-55 | 9 | 100 |

**TABLE IV**

| **Insecticidal and Acaricidal Evaluation of N-Arylamidrazones** | | | |
|---|---|---|---|
| | % Mortality | | |
| Compound (Ex. No.) | Armyworm¹ (300 ppm) | 2-Spotted Mite² (300 ppm) | Corn Rootworm³ (50 kg/ha) |
| 85 | 0 | 0 | 100 |
| 86 | 100 | 0 | 80 |
| 87 | 40 | 90 | 100 |
| 88 | --- | --- | --- |
| 89 | 0 | 0 | 100 |
| 90 | 0 | 0 | 20 |
| 91 | 0 | 80 | 100 |
| 92 | 0 | 0 | 100 |
| 93 | 0 | 0 | 100 |
| 94 | --- | 80 | 100 |
| 95 | 80 | 0 | 100 |
| 96 | 100 | 40 | 80 |
| 97 | 0 | 0 | 100 |
| 98 | 40 | 0 | 40 |
| 100 | 0 | 40 | 0 |
| 101 | 0 | 0 | 60 |
| 102 | 0 | 60 | 100 |
| 103 | 40 | 0 | 100 |
| 104 | 0 | 90 | 50 |
| 105 | 20 | 0 | 90 |
| 106 | 40 | 0 | 100 |
| 107 | --- | --- | 100 |
| 108 | 90 | 50 | 100 |
| 109 | 0 | 0 | 50 |
| 110 | 0 | 0 | 100 |
| 111 | 100 | 40 | 90 |
| 112 | 40 | 100 | 20 |
| 113 | 20 | 100 | 100 |
| 114 | 40 | 100 | 100 |
| 115 | 0 | 0 | 100 |
| 116 | 20 | 50 | 100 |
| 117 | 20 | 0 | 100 |
| 118 | 50 | 70 | 100 |
| 119 | 100 | 50 | 90 |
| 120 | --- | 30 | 20 |
| 121 | 80 | 40 | 100 |
| 122 | 0 | 0 | 40 |
| 123 | 0 | 0 | 60 |
| 124 | 50 | 80 | 100 |
| 125 | 0 | 30 | 100 |
| 126 | 0 | 80 | 90 |
| 128 | 0 | 0 | 30 |
| 129 | 100 | 40 | 0 |
| 130 | 80 | 80 | 100 |
| 131 | 70 | 0 | 100 |
| 132 | -- | 40 | 100 |
| 133* | -- | 0 | 0 |
| 134* | 0 | 30 | 0 |
| 135 | 0 | 0 | 0 |
| 136 | 0 | 70 | 100 |
| 137 | 0 | 0 | 100 |
| 138 | 0 | 0 | 100 |
| 139 | 0 | 70 | 100 |
| 140 | 0 | 0 | 50 |
| 141 | 100 | 0 | 0 |
| 142 | 0 | 0 | 100 |
| 143 | 0 | 0 | 100 |
| 144 | 0 | 0 | 100 |
| 145 | 0 | 0 | 100 |
| 146 | 0 | 0 | 100 |
| 147 | 0 | 0 | 100 |
| 148 | 50 | 0 | 100 |
| 149 | 100 | 80 | 80 |
| 150 | 0 | 60 | 100 |
| 152 | 80 | 0 | 100 |
| 153 | 100 | 0 | 100 |
| 156 | --- | 0 | 100 |
| 157 | 0 | 0 | 100 |
| 158 | 40 | 0 | 100 |
| 159 | 0 | 0 | 100 |
| 160 | 0 | 0 | 100 |
| 161 | 0 | 0 | --- |
| 162 | 0 | 100 | 100 |
| 163 | 0 | 0 | 100 |
| 164 | 0 | 0 | 100 |
| 167 | 0 | 0 | 100 |
| 168 | 0 | 80 | 90 |
| 169 | 0 | 0 | 100 |

| | | | |
|---|---|---|---|
| ¹Armyworm is 3rd instar larvae, southern armyworm | | | |
| ²2-Spotted Mite is 2-spotted spider mite (OP-resistant) | | | |
| ³Corn Rootworm is 3rd instar southern corn rootworm | | | |
| * Comparative Example | | | |

**TABLE V**

| **Insecticidal and Acaricidal Evaluation of N-Arylhydrazides** | | | |
|---|---|---|---|
| Compound (Ex. No.) | Armyworm¹ (300 ppm) | 2-Spotted Mite² (300 ppm) | Corn Rootworm³ (50 kg/ha) |
| 1 | 8 | 0 | 9 |
| 2 | 0 | 0 | 7 |
| 3 | --- | --- | 9 |
| 4 | 0 | 0 | 7 |
| 5 | 0 | 0 | 8 |
| 6 | 0 | 0 | 0 |
| 7* | 0 | 0 | 0 |
| 8 | 5 | 0 | 8 |
| 9 | 0 | 0 | 0 |
| 10 | 1 | 9 | 3 |
| 11 | 1 | 0 | 9 |
| 12 | 4 | 0 | 4 |
| 13 | 0 | 9 | 3 |
| 14 | 7 | 0 | 7 |
| 15 | 9 | 0 | 3 |
| 16 | 0 | 0 | 0 |
| 17 | 1 | 3 | 0 |
| 18 | 2 | 0 | 6 |
| 19 | 9 | 0 | 0 |
| 20 | 0 | 0 | 0 |
| 21 | 0 | 0 | 7 |
| 22 | 0 | 0 | 0 |
| 23 | 0 | 0 | 0 |
| 24 | 0 | 0 | 0 |
| 25 | 9 | 0 | 8 |
| 26 | 0 | 0 | 0 |
| 27 | 4 | 0 | 6 |
| 28 | 2 | 0 | 0 |
| 29 | 3 | 0 | 0 |
| 30 | 0 | 2 | 4 |
| 31 | 0 | 0 | 0 |
| 32 | 1 | 0 | 0 |
| 33 | 0 | 0 | 0 |
| 34 | 8 | 0 | 2 |
| 35 | 5 | 0 | 0 |
| 36 | 8 | 0 | 0 |
| 37 | 4 | 0 | 0 |
| 39 | 0 | 0 | 0 |
| 40 | 9 | 0 | 9 |
| 41 | 3 | 0 | 9 |
| 42 | 0 | 2 | 4 |

| | | | |
|---|---|---|---|
| ¹Armyworm is 3rd instar larvae, southern armyworm | | | |
| ²2-Spotted Mite is 2-spotted spider mite (OP-resistant) | | | |
| ³Corn Rootworm is 3rd instar southern corn rootworm | | | |
| * Comparative Example | | | |

**TABLE VI**

| **Insecticidal and Acaricidal Evaluation of Substituted N-Arylhydrazinoyl Halides** | | | |
|---|---|---|---|
| | % Mortality | | |
| Compound (Comp. Ex. No.) | Armyworm¹ (300 ppm) | 2-Spotted Mite² (300 ppm) | Corn Rootworm³ (50 kg/ha) |
| 78 | 90 | 90 | 0 |
| 54 | 80 | 100 | 0 |
| 58 | 0 | 0 | 0 |
| 59 | 0 | 100 | 0 |
| 64 | --- | 90 | 100 |
| 66 | 80 | 100 | 20 |
| 71 | 90 | 90 | 30 |
| 73 | 50 | 100 | 0 |
| 77 | 100 | 90 | 80 |
| 79 | 100 | 100 | 100 |

| | | | |
|---|---|---|---|
| ¹Armyworm is 3rd instar larvae, southern armyworm | | | |
| ²2-Spotted Mite is 2-spotted spider mite (OP-resistant) | | | |
| ³Corn Rootworm is 3rd instar southern corn rootworm | | | |

## Claims

1. A method for the control of insect or acarid pests which comprises contacting said pests or their food supply, habitat or breeding grounds with a pesticidally effective amount of a compound having the structure 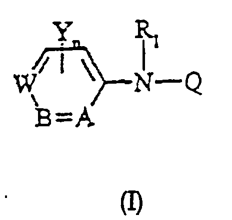 wherein
A is C-R₄ or N;
B is C-R₅ or N;
W is C-R₆ or N, with the proviso that at least one of A, B or W must be other than N; and with the further proviso that when all of A, B and W, are other than N, then R cannot be phenyl or substituted phenyl;
Y is hydrogen, halogen, CN, NO₂, C₁-C₆ alkyl, C₁-C₈haloalkyl, C₁-C₆alkoxy or C₁-C₆haloalkoxy;
n is an integer of 0, 1 or 2;
Q is 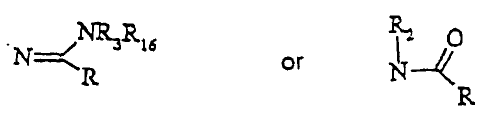
R is hydrogen,
C₁-C₁₀alkyl, optionally substituted with one or more halogens, C₃-C₆ cycloalkyl, C₁-C₄alkoxy, C₁-C₄haloalkoxy, (C₁-C₄alkyl)SOₓ, (C₁-C₄haloalkyl)SOₓ,
phenyl, optionally substituted with one to three halogen, C₁-C₄alkyl, C₁-C₄haloalkyl, C₁-C₄alkoxy, C₁-C₄haloalkoxy, (C₁-C₄ alkyl)SOₓ, (C₁-C₄haloalkyl)SOₓ, NO₂ or CN groups, or
phenoxy, optionally substituted with one to three halogen, C₁-C₄alkyl, C₁-C₄ haloalkyl, C₁-C₄alkoxy, C₁-C₄haloalkoxy, (C₁-C₄alkyl)SOₓ, (C₁-C₄ haloalkyl)SOₓ, NO₂ or CN groups,
C₃-C₁₂ cycloalkyl, optionally substituted with one or more halogens, C₁-C₆,alkyl, C₁-C₆,haloalkyl, C₁-C₄alkoxy, C₁-C₄haloalkoxy, (C₁-C₄alkyl)SOₓ, (C₁-C₄haloalkyl)SOₓ,
phenyl, optionally substituted with one to three halogen, C₁-C₄alkyl, C₁-C₄haloalkyl, C₁-C₄alkoxy, C₁-C₄haloalkoxy, NO₂ or CN groups, or
phenoxy, optionally substituted with one to three halogen, C₁-C₄alkyl, C₁-C₄ haloalkyl, C₁-C₄alkoxy, C₁-C₄haloalkoxy, NO₂ or CN groups, or
phenyl, optionally substituted with one or more halogen, C₁-C₄alkyl, C₁-C₄haloalkyl, C₁-C₄alkoxy, C₁-C₄haloalkoxy, NO₂ or CN groups;
R₁ and R₂ are each independently hydrogen or C₁-C₄alkyl;
R₃ and R₁₆ are each independently hydrogen,
C₁-C₁₀alkyl, optionally substituted with one or more halogen,
hydroxy, C₁-C₄alkoxy, (C₁-C₄alkyl)SOₓ, CONR₇R_{8,} CO₂R₉, R₁₀, R₁₁, C₃-C₆cycloalkyl, optionally substituted with one to three halogen, C₁-C₄alkyl, C₁-C₄haloalkyl, C₁-C₄alkoxy, C₁-C₄haloalkoxy, NO₂ or CN groups,
phenyl, optionally substituted with one or more halogen, C₁-C₄alkyl, C₁-C₄ haloalkyl, C₁-C₄alkoxy, C₁-C₄haloalkoxy, NO₂ or CN groups, or
pyridyl, optionally substituted with one or more halogen, C₁-C₄alkyl, C₁-C₄ haloalkyl, C₁-C₄alkoxy, C₁-C₄haloalkoxy, NO₂ or CN groups,
C₃-C₁₀alkenyl, optionally substituted with one or more halogen,
hydroxy, C₁-C₄alkoxy, (C₁-C₄alkyl)SOₓ, CONR₇R₈, CO₂R₉, R₁₀, R₁₁, C₃-C₆cycloalkyl, optionally substituted with one to three halogen, C₁-C₄alkyl, C₁-C₄ haloalkyl, C₁-C₄alkoxy, C₁-C₄haloalkoxy, NO₂ or CN groups,
phenyl, optionally substituted with one or more halogen, C₁-C₄alkyl, C₁-C₄ haloalkyl, C₁-C₄alkoxy, C₁-C₄haloalkoxy or CN groups, or
pyridyl, optionally substituted with one or more halogen,C₁-C₄alkyl, C₁-C₄haloalkyl, C₁-C₄alkoxy, C₁-C₄haloalkoxy, NO₂ or CN groups,
C₃-C₁₀alkynyl, optionally substituted with one or more halogen, hydroxy,
C₁-C₄alkoxy, (C₁-C₄alkyl)SOₓ, CONR₇R₈, CO₂R₉,
C₃-C₆cycloalkyl, optionally substituted with one to three halogen, C₁-C₄alkyl, C₁-C₄ haloalkyl, C₁-C₄alkoxy, C₁-C₄haloalkoxy, NO₂ or CN groups,
phenyl, optionally substituted with one or more halogen, C₁-C₄alkyl, C₁-C₄haloalkyl, C₁-C₄alkoxy, C₁-C₄haloalkoxy or CN groups, or
pyridyl, optionally substituted with one or more halogen, C₁-C₄alkyl, C₁-C₄haloalkyl, C₁-C₄alkoxy, C₁-C₄haloalkoxy, NO₂ or CN groups,
C₃-C₁₂cycloalkyl, optionally substituted with one or more halogen,
hydroxy, C₁-C₄alkoxy, (C₁-C₄alkyl) SOₓ, CO₂R₇R₈, CO₂R₉, R₁₀, R₁₁, C₃-C₆cycloalkyl, optionally substituted with one to three halogen, C₁-C₄alkyl, C₁-C₄ haloalkyl, C₁-C₄alkoxy, C₁-C₄ haloalkoxy, NO₂ or CN groups,
phenyl, optionally substituted with one or more halogen, C₁-C₄alkyl, C₁-C₄haloalkyl, C₁-C₄alkoxy, C₁-C₄haloalkoxy, or CN groups, or
pyridyl, optionally substituted with one or more halogen, C₁-C₄alkyl, C₁-C₄haloalkyl, C₁-C₄alkoxy, C₁-C₄haloalkoxy, NO₂ or CN groups or
R₃ and R₁₆ may be taken together to form a ring represented by the structure 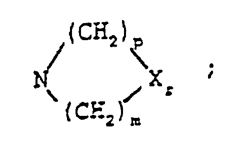
R₄, R₅ and R₆ are each independently hydrogen, halogen, CN, NO₂, (C₁- C₄alkyl)SOₓ, (C₁-C₄haloalkyl)SOₓ, C₁-C₆alkyl, C₁-C₆haloalkyl, C₁-C₆alkoxy or C₁-C₆haloalkoxy;
R₇, R₈ and R₉ are each independently hydrogen or C₁-C₄ alkyl;
R₁₀ is NR₁₂R₁₃, 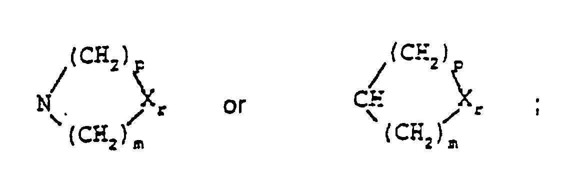
R₁₁ is 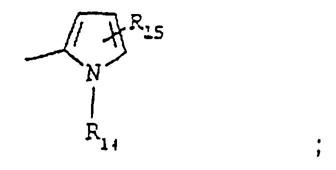
R₁₂, R₁₃, R₁₄ and R₁₅ are each independently hydrogen or C₁-C₄alkyl;
X is O, S or NR₁₄;
r is an integer of 0 or 1;
p and m are each independently an integer of 0, 1, 2 or 3, with the proviso that the sum of p + m + r must be 4, 5 or 6;
x is an integer of 0, 1 or 2; or
the acid addition salts thereof.

2. The method according to claim 1 wherein Q is 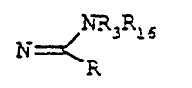 and R, R₃, R₁₆ are defined as in claim 1.

3. The method according to claim 2 wherein A is C-R₄, B is CH, W is C-R₆, Y is halogen, n is 1 and R₁ is hydrogen, R₄ and R₆ are each independently halogen or C₁-C₆alkyl substituted with one or more halogens, and R, R₃ and R₁₆ are each independently hydrogen or C₁-C₁₀alkyl.

4. The method according to claim 1 wherein Q is 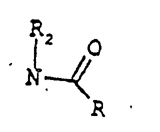 and R and R₂ are as defined in claim 1.

5. The method according to claim 4 wherein R₁ and R₂ are hydrogen, R is C₁-C₆alkyl, A is C-R₃, B is C-R₄, W is C-R₅ Y is halogen, n is 1, R₃ is halogen, R₄ is hydrogen and R₅ is C₁-C₆ alkyl substituted with one or more halogens.

6. The method according to claim 5 wherein the compound is 2,2-dimethylpropionic acid, 2-(2,6-dichloro-a,a,a-trifluoro-p-tolyl)hydrazide.

7. A compound having the structure 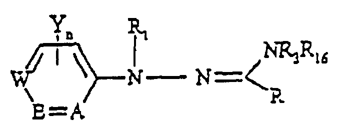 wherein A, B, W, Y, n, R₁, R₃, and R₁₆, are as defined in claim 1 with the proviso that
(a) when one of A, B and W is N, then at least one of Y, R₄, R₅ and R₆ must be other than C₁-C₁₀alkyl; and
(b) when all of A, B or W are other than N, then one of R₄ or R₆ must be other than NO₂ and R₄ and at least one of R₃ or R₁₆ must be other than hydrogen.

8. The compound according to claim 7 having the structure 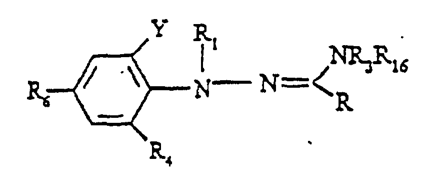 wherein
R is C₁-C₁₀alkyl;
R₁ is hydrogen or C₁-C₄alkyl;
R₃ is C₁-C₁₀alkyl;
R₁₆ is hydrogen or C₁-C₁₀alkyl; and
R₄, R₆ and Y are each independently hydrogen, halogen, CN, C₁-C₆alkyl, C₁-C₄haloalkyl, C₁-C₆alkoxy or C₁-C₆haloalkoxy.

9. The compound according to claim 8, N-ethyl-2,2-dimethylpropionamide, 2-(2,6-dichloro-α,α,α-trifluoro-p-tolyl)hydrazone.

10. A process for the preparation of a compound having the structure 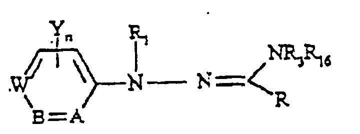 wherein A, B, W, Y, n, R, R₁, R₃ and R₁₆ are as defined in claim 7 which comprises reacting a compound having the structure 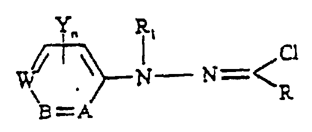 with at least one molar equivalent of an amine compound, HNR₃R₁₆.

11. A composition for controlling insect or acarid pests which comprises an inert liquid or solid carrier and a pesticidally effective amount of a compound of formula I 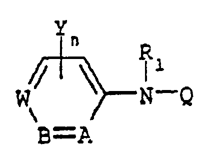 wherein Q is 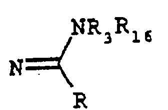 and A, B, W, Y, n, R_{1,} R₃ and R₁₆ are as defined in claim 7.

12. The composition according to claim 11 wherein the formula I compound is as defined in claim 8.

13. Use of a composition as defined in claims 11 or 12 for controlling insect or acarid pests.

## Patentansprüche

1. Verfahren zur Bekämpfung von Schädlingen (Insekten oder Akarinen), bei dem man die Schädlinge oder ihre Nahrungsmittelquellen, ihren Lebensraum oder ihre Brutstätten mit einer pestizid wirksamen Menge einer Verbindung mit der Struktur 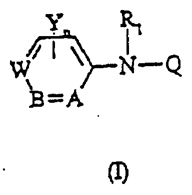 in welcher
A für C-R₄ oder N steht;
B für C-R₅ oder N steht;
W für C-R₆ oder N steht, mit der Maßgabe, daß wenigstens einer der Reste A, B oder W nicht für N steht; und mit der weiteren Maßgabe, daß wenn alle der Reste A, B und W nicht für N stehen, R nicht für Phenyl oder substituiertes Phenyl steht;
Y für Wasserstoff, Halogen, CN, NO₂, C₁-C₆-Alkyl, C₁-C₆-Halogenalkyl, C₁-C₆-Alkoxy oder C₁-C₆-Halogenalkoxy steht;
n für eine ganze Zahl 0, 1 oder 2 steht;
Q für 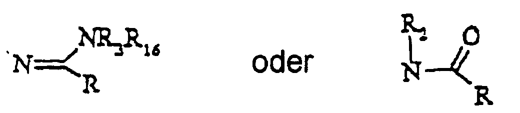
steht;
R für Wasserstoff,
C₁-C₁₀-Alkyl, welches gegebenenfalls durch ein oder mehrere Halogene, C₃-C₆-Cycloalkyl, C₁-C₄-Alkoxy, C₁-C₄-Halogenalkoxy, (C₁-C₄-Alkyl)SOₓ, (C₁-C₄-Halogenalkyl)SOₓ,
Phenyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, (C₁-C₄-Alkyl)SOₓ-, (C₁-C₄-Halogenalkyl)SOₓ-, NO₂- oder CN-Gruppen substituiert ist, oder
Phenoxy, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, (C₁-C₄-Alkyl)SOₓ-, (C₁-C₄-Halogenalkyl)SOₓ-, NO₂- oder CN-Gruppen substituiert ist, substituiert ist,
C₃-C₁₂-Cycloalkyl, welches gegebenenfalls durch ein oder mehrere Halogene, C₁-C₆-Alkyl, C₁-C₆-Halogenalkyl, C₁-C₄-Alkoxy, C₁-C₄-Halogenalkoxy, (C₁-C₄-Alkyl)SOₓ, (C₁-C₄-Halogenalkyl)SOₓ,
Phenyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, oder
Phenoxy, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, substituiert ist, oder
Phenyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, steht;
R₁ und R₂ jeweils unabhängig voneinander für Wasserstoff oder C₁-C₄-Alkyl stehen;
R₃ und R₁₆ jeweils unabhängig voneinander für Wasserstoff,
C₁-C₁₀-Alkyl, welches gegebenenfalls durch ein oder mehrere Halogene, Hydroxyl, C₁-C₄-Alkoxy, (C₁-C₄-Alkyl)SOₓ, CONR₇R₈, CO₂R₉, R₁₀, R₁₁,
C₃-C₈-Cycloalkyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist,
Phenyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, oder
Pyridyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, substituiert ist,
C₃-C₁₀-Alkenyl, welches gegebenenfalls durch ein oder mehrere Halogene, Hydroxyl, C₁-C₄-Alkoxy, (C₁-C₄-Alkyl)SOₓ, CONR₇R₈, CO₂R₉, R₁₀, R₁₁,
C₃-C₆-Cycloalkyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist,
Phenyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy- oder CN-Gruppen substituiert ist, oder
Pyridyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, substituiert ist,
C₃-C₁₀-Alkinyl, welches gegebenenfalls durch ein oder mehrere Halogene, Hydroxyl, C₁-C₄-Alkoxy, (C₁-C₄-Alkyl)SOₓ, CONR₇R₈, CO₂R₉,
C₃-C₆-Cycloalkyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist,
Phenyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy- oder CN-Gruppen substituiert ist, oder
Pyridyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, substituiert ist,
C₃-C₁₂-Cycloalkyl, welches gegebenenfalls durch ein oder mehrere Halogene, Hydroxyl, C₁-C₄-Alkoxy, (C₁-C₄-Alkyl)SOₓ, CONR₇R₈, CO₂R₉, R₁₀, R₁₁,
C₃-C₆-Cycloalkyl, welches gegebenenfalls durch ein bis drei Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist,
Phenyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy- oder CN-Gruppen substituiert ist, oder
Pyridyl, welches gegebenenfalls durch ein oder mehrere Halogen-, C₁-C₄-Alkyl-, C₁-C₄-Halogenalkyl-, C₁-C₄-Alkoxy-, C₁-C₄-Halogenalkoxy-, NO₂- oder CN-Gruppen substituiert ist, substituiert ist, stehen oder
R₃ und R₁₆ zusammen einen durch die Struktur 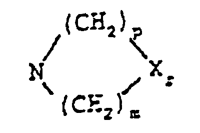 wiedergegebenen Ring bilden können;
R₄, R₅ und R₆ jeweils unabhängig voneinander für Wasserstoff, Halogen, CN, NO₂, (C₁-C₄-Alkyl)SOₓ, (C₁-C₄-Halogenalkyl) SOₓ, C₁-C₆-Alkyl, C₁-C₆-Halogenalkyl, C₁-C₆-Alkoxy oder C₁-C₆-Halogenalkoxy stehen;
R₇, R₈ und R₉ jeweils unabhängig für Wasserstoff oder C₁-C₄-Alkyl stehen;
R₁₀ für NR₁₂R₁₃, 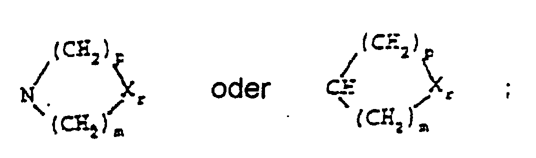 steht;
R₁₁ für 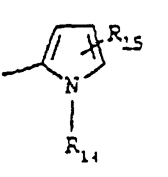 steht;
R₁₂, R₁₃, R₁₄ und R₁₅ jeweils unabhängig voneinander für Wasserstoff oder C₁-C₄-Alkyl stehen;
X für O, S oder NR₁₄ steht;
r für eine ganze Zahl 0 oder 1 steht;
p und m jeweils unabhängig voneinander für ganze Zahlen 0, 1, 2 oder 3 stehen,
mit der Maßgabe, daß die Summe von p + m + r 4, 5 oder 6 betragen muß;
x für eine ganze Zahl 0, 1 oder 2 steht; oder
deren Säureadditionssalzen in Kontakt bringt.

2. Verfahren nach Anspruch 1, wobei Q für 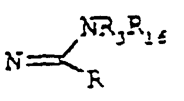 steht und R, R₃ und R₁₆ wie nach Anspruch 1 definiert sind.

3. Verfahren nach Anspruch 2, wobei A für C-R₄, B für CH, W für C-R₆, Y für Halogen, n für 1 und R₁ für Wasserstoff steht und R₄ und R₆ jeweils unabhängig voneinander für Halogen oder durch ein oder mehrere Halogene substituiertes C₁-C₆-Alkyl und R, R₃ und R₁₆ jeweils unabhängig voneinander für Wasserstoff oder C₁-C₁₀-Alkyl stehen.

4. Verfahren nach Anspruch 1, wobei Q für 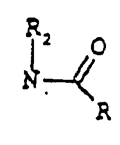 steht und R und R₂ wie in Anspruch 1 definiert sind.

5. Verfahren nach Anspruch 4, wobei R₁ und R₂ für Wasserstoff stehen, R für C₁-C₆-Alkyl, A für C-R₃, B für C-R₄, W für C-R₅, Y für Halogen, n für 1, R₃ für Halogen, R₄ für Wasserstoff und R₅ für durch ein oder mehrere Halogene substituiertes C₁-C₆-Alkyl steht.

6. Verfahren nach Anspruch 5, wobei es sich bei der Verbindung um 2,2-Dimethylpropionsäure-2-(2,6-dichlor-α,α,α-trifluor-p-tolyl)hydrazid handelt.

7. Verbindungen mit der Struktur 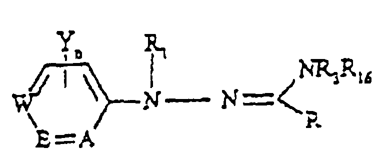 in welcher A, B, W, Y, n, R₁, R₃ und R₁₆ wie in Anspruch 1 definiert sind, mit der Maßgabe, daß
(a) wenn einer der Reste A, B und W für N steht, wenigstens einer der Reste Y, R₄, R₅ und R₆ nicht für C₁-C₁₀-Alkyl steht; und
(b) wenn alle der Reste A, B und W nicht für N stehen, einer der Reste R₄ und R₆ nicht für NO₂ steht und R₄ und wenigstens einer der Reste R₃ und R₁₆ nicht für Wasserstoff steht.

8. Verbindungen nach Anspruch 7 mit der Struktur 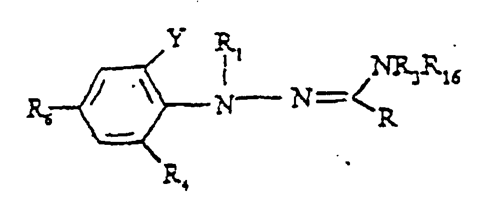 in welcher
R für C₁-C₁₀-Alkyl steht;
R₁ für Wasserstoff oder C₁-C₄-Alkyl steht;
R3 für C₁-C₁₀-Alkyl steht;
R₁₆ für Wasserstoff oder C₁-C₁₀-Alkyl steht; und
R₄, R₆ und Y jeweils unabhängig voneinander für Wasserstoff, Halogen, CN, C₁-C₆-Alkyl, C₁-C₄-Halogenalkyl, C₁-C₆-Alkoxy oder C₁-C₆-Halogenalkoxy stehen.

9. Verbindung nach Anspruch 8, bei der es sich um N-Ethyl-2,2-dimethylpropionsäureamid-2-(2,6-dichlor-α,α,α-trifluor-p-tolyl)hydrazon handelt.

10. Verfahren zur Darstellung einer Verbindung mit der Struktur 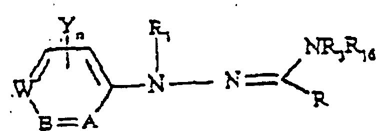 in welcher A, B, W, Y, n, R, R₁, R₃ und R₁₆ wie in Anspruch 7 definiert sind, bei dem man eine Verbindung mit der Struktur 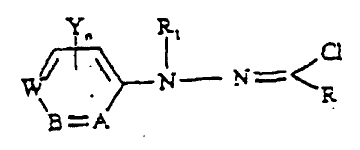 mit wenigstens einem Moläquivalent einer Aminverbindung HNR₃R₁₆ umsetzt.

11. Zusammensetzung zur Bekämpfung von Schädlinger (Insekten oder Akarinen), enthaltend einen inerten flüssigen oder festen Trägerstoff und eine pestizid wirksame Menge einer Verbindung der Formel I 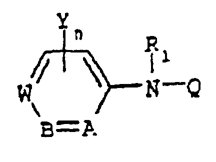 in welcher Q für 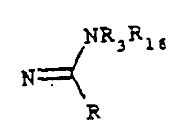 steht und A, B, W, Y, n, R₁, R₃ und R₁₆ wie in Anspruch 7 definiert sind.

12. Zusammensetzung nach Anspruch 11, wobei die Verbindung der Formel I wie in Anspruch 8 definiert ist.

13. Verwendung einer wie in Anspruch 11 oder 12 definierten Zusammensetzung zur Bekämpfung von Schädlingen (Insekten oder Akarinen).

## Revendications

1. Procédé de lutte contre des insectes et acariens nuisibles, qui consiste à mettre en contact lesdits nuisibles ou leur source de nourriture, leur habitat ou leurs sites de reproduction avec une quantité à effet pesticide d'un composé ayant la structure 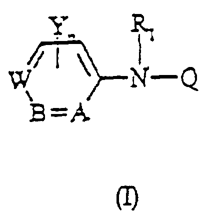 dans laquelle
A est C-R₄ ou N ;
B est C-R₅ ou N ;
W est C-R₆ ou N, avec la condition qu'au moins l'un de A, B et W soit autre chose que N ; et avec la condition supplémentaire que si A, B et W sont tous autre chose que N, alors R ne soit pas un groupe phényle ou phényle substitué ;
Y est l'hydrogène, un halogène, CN, NO₂, un groupe alkyle en C₁-C₆, halogénoalkyle en C₁-C₆, alcoxy en C₁-C₆ ou halogénoalcoxy en C₁-C₆ ;
n est le nombre entier 0, 1 ou 2 ;
Q est 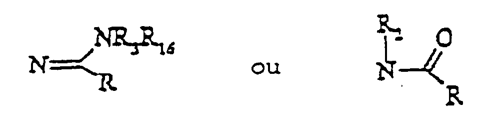
R est l'hydrogène,
un groupe alkyle en C₁-C₁₀ facultativement substitué par un ou plusieurs groupes halogéno, cycloalkyle en C₃-C₆, alcoxy en C₁-C₄, halogénoalcoxy en C₁-C₄, (alkyle en C₁-C₄)SOₓ, (halogénoalkyle en C₁-C₄)SOₓ,
phényle, facultativement substitué par un à trois atomes groupes halogéno, alkyle en C₁-C₄, halogénoalkyle en C₁-C₄, alcoxy en C₁-C₄, halogénoalcoxy en C₁-C₄, (alkyle en C₁-C₄)SOₓ, (halogénoalkyle en C₁-C₄)SOₓ, NO₂ ou CN, ou
phénoxy, facultativement substitué par un à trois groupes halogéno, alkyle en C₁-C₄, halogénoalkyle en C₁-C₄, alcoxy en C₁-C₄, halogénoalcoxy en C₁-C₄, (alkyle en C₁-C₄)SOₓ, (halogénoalkyle en C₁-C₄)SOₓ, NO₂ ou CN,
un groupe cycloalkyle en C₃-C₁₂, facultativement substitué par un ou plusieurs groupes halogéno, alkyle en C₁-C₆, halogénoalkyle en C₁-C₆, alcoxy en C₁-C₄, halogénoalcoxy en C₁-C₄, (alkyle en C₁-C₄)SOₓ, (halogénoalkyle en C₁-C₄)SOₓ,
phényle, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN, ou
phénoxy, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN, ou
un groupe phényle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN ;
R₁ et R₂ sont chacun indépendamment de l'hydrogène ou un groupe alkyle en C₁-C₄ ;
R₃ et R₁₆ sont chacun indépendamment l'hydrogène,
un groupe alkyle en C₁-C₁₀, facultativement substitué par un ou plusieurs groupes halogéno, hydroxyle, alcoxy en C₁-C₄, (alkyle en C₁-C₄)SOₓ, CONR₇R₈, CO₂R₉, R₁₀, R₁₁,
cycloalkyle en C₃-C₈, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN,
phényle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN, ou
pyridyle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN,
un groupe alcényle en C₃-C₁₀, facultativement substitué par un ou plusieurs groupes halogéno, hydroxyle, alcoxy en C₁-C₄, (alkyle en C₁-C₄)SOₓ, CONR₇R₈, CO₂R₉, R₁₀, R₁₁,
cycloalkyle en C₃-C₆, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN,
phényle, facultativement substitué par un ou plusieurs groupes halogéno, alkyle en C₁-C₄, halogénoalkyle en C₁-C₄, alcoxy en C₁-C₄, halogénoalcoxy en C₁-C₄ ou CN, ou
pyridyle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN,
un groupe alcynyle en C₃-C₁₀, facultativement substitué par un ou plusieurs groupes halogéno, hydroxyle, alcoxy en C₁-C₄, (alkyle en C₁-C₄)SOₓ, CONR₇R₈, CO₂R₉,
cycloalkyle en C₃-C₆, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN,
phényle, facultativement substitué par un ou plusieurs groupes halogéno, alkyle en C₁-C₄, halogénoalkyle en C₁-C₄, alcoxy en C₁-C₄, halogénoalcoxy en C₁-C₄ ou CN, ou
pyridyle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN,
un groupe cycloalkyle en C₃-C₁₂, facultativement substitué par un ou plusieurs groupes halogéno, hydroxyle, alcoxy en C₁-C₄, (alkyle en C₁-C₄)SOₓ, CO₂R₇R₈, CO₂R₉, R₁₀, R₁₁,
cycloalkyle en C₃-C₆, facultativement substitué par un à trois groupes halogéno, alkyle en C₁-C₄, halogénoalkyle en C₁-C₄, alcoxy en C₁-C₄, halogénoalcoxy en C₁-C₄, NO₂ ou CN,
phényle, facultativement substitué par un ou plusieurs groupes halogéno, alkyle en C₁-C₄, halogénoalkyle en C₁-C₄, alcoxy en C₁-C₄, halogénoalcoxy en C₁-C₄ ou CN, ou
pyridyle, facultativement substitué par un ou plusieurs groupes halogéno, alkyle en C₁-C₄, halogénoalkyle en C₁-C₄, alcoxy en C₁-C₄, halogénoalcoxy en C₁-C₄, NO₂ ou CN, ou bien
R₃ et R₁₆ peuvent être pris ensemble pour former un cycle représenté par la structure 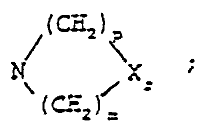
R₄, R₅ et R₆ sont chacun indépendamment de l'hydrogène, un halogène, CN, NO₂, un groupe (alkyle en C₁-C₄)SOₓ, (halogénoalkyle en C₁-C₄)SOₓ, alkyle en C₁-C₆, halogénoalkyle en C₁-C₆, alcoxy en C₁-C₆ ou halogénoalcoxy en C₁-C₆ ;
R₇, R₈ et R₉ sont chacun indépendamment l'hydrogène ou un groupe alkyle en C₁-C₄ ;
R₁₀ est NR₁₂R₁₃, 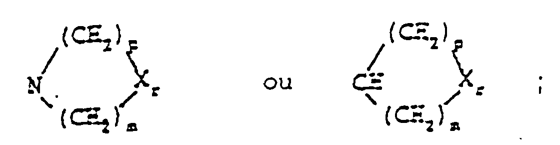
R₁₁ est 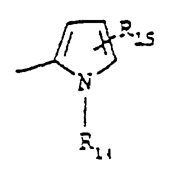
R₁₂, R₁₃, R₁₄ et R₁₅ sont chacun indépendamment l'hydrogène ou un groupe alkyle en C₁-C₄ ;
X est O, S ou NR₁₄ ;
r est le nombre entier 0 ou 1 ;
p et m sont chacun indépendamment le nombre entier 0, 1, 2 ou 3, avec la condition que la somme p + m + r soit égale à 4, 5 ou 6 ;
x est le nombre entier 0, 1 ou 2 ;
ou d'un de ses sels d'addition d'acide.

2. Procédé selon la revendication 1, dans lequel Q est 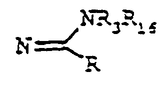 et R, R₃ et R₁₆ sont tels que définis dans la revendication 1.

3. Procédé selon la revendication 2, dans lequel A est C-R₄, B est CH, W est C-R₆, Y est un halogène, n est 1 et R₁ est l'hydrogène, R₄ et R₆ sont chacun indépendamment un halogène ou un groupe alkyle en C₁-C₆ substitué par un ou plusieurs halogènes, et R, R₃ et R₁₆ sont chacun indépendamment l'hydrogène ou un groupe alkyle en C₁-C₁₀.

4. Procédé selon la revendication 1, dans lequel
Q est 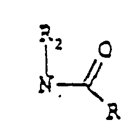
et R et R₂ sont tels que définis dans la revendication 1.

5. Procédé selon la revendication 4, dans lequel R₁ et R₂ sont l'hydrogène, R est un groupe alkyle en C₁-C₆, A est C-R₃, B est C-R₄, W est C-R₅, Y est un halogène, n est 1, R₃ est un halogène, R₄ est l'hydrogène et R₅ est un groupe alkyle en C₁-C₆ substitué par un ou plusieurs halogènes.

6. Procédé selon la revendication 5, dans lequel le composé est le 2-(2,6-dichloro-α,α,α-trifluoro-*p*-tolyl)-hydrazide d'acide 2,2-diméthylpropionique.

7. Composé ayant la structure 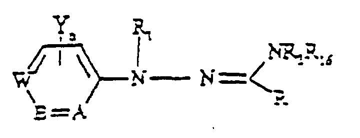 dans laquelle A, B, W, Y, n, R₁, R₃ et R₁₆ sont tels que définis dans la revendication 1, à condition que
(a) lorsque l'un de A, B et W est N, alors au moins l'un de Y, R₄, R₅ et R₆ soit autre chose qu'un groupe alkyle en C₁-C₁₀ ;
(b) lorsque A, B et W sont tous autre chose que N, alors l'un de R₄ et R₆ soit autre chose que NO₂ et R₄ et au moins l'un de R₃ et R₁₆ soient autre chose que l'hydrogène.

8. Composé selon la revendication 7, ayant la structure 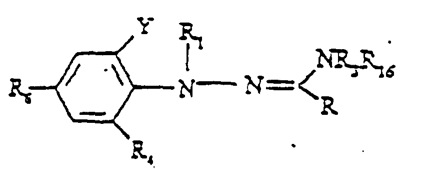 dans laquelle
R est un groupe alkyle en C₁-C₁₀ ;
R₁ est l'hydrogène ou un groupe alkyle en C₁-C₄ ;
R3 est un groupe alkyle en C₁-C₁₀ ;
R₁₆ est l'hydrogène ou un groupe alkyle en C₁-C₁₀ ; et
R₄, R₆ et Y sont chacun indépendamment l'hydrogène, un halogène, CN, un groupe alkyle en C₁-C₆, halogénoalkyle en C₁-C₄, alcoxy en C₁-C₆ ou halogénoalcoxy en C₁-C₆.

9. Composé selon la revendication 8, la 2-(2,6-di-chloro-α,α,α-trifluoro-p-tolyl)hydrazone de N-éthyl-2,2-diméthylpropionamide.

10. Procédé pour la préparation d'un composé ayant la structure 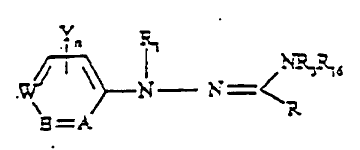 dans laquelle A, B, W, Y, n, R, R₁, R₃ et R₁₆ sont tels que définis dans la revendication 7, qui comprend la réaction d'un composé ayant la structure 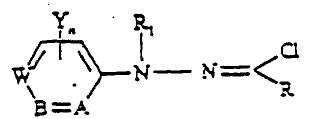 avec au moins un équivalent molaire d'une amine, HNR₃R₁₆.

11. Composition pour combattre des insectes ou acariens nuisibles, qui comprend un support liquide ou solide inerte et une quantité à effet pesticide d'un composé de formule I 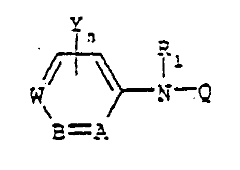 dans laquelle Q est 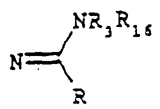 et A, B, W, Y, n, R₁, R₃ et R₁₆ sont tels que définis dans la revendication 7.

12. Composition selon la revendication 11, dans laquelle le composé de formule I est tel que défini dans la revendication 8.

13. Utilisation d'une composition telle que définie dans les revendications 11 ou 12, pour combattre des insectes ou acariens nuisibles.
